(19) Europäisches Patentamt
European Patent Office
Office européen des brevets

(11) Numéro de publication : **0 446 140 A1**

# (12) DEMANDE DE BREVET EUROPEEN

(21) Numéro de dépôt : **91400644.0**

(22) Date de dépôt : **08.03.91**

(51) Int. Cl.[5] : **C07D 495/14**, A61K 31/55, // (C07D495/14, 333:00, 207:00, 243:00)

(30) Priorité : **08.03.90 FR 9002934**

(43) Date de publication de la demande : **11.09.91 Bulletin 91/37**

(84) Etats contractants désignés : **AT BE CH DE DK ES FR GB GR IT LI LU NL SE**

(71) Demandeur : **ADIR ET COMPAGNIE**
**1 rue Carle Hébert**
**F-92415 Courbevoie Cédex (FR)**

(72) Inventeur : **Rault, Sylvain**
**Route de St Pierre sur Dives**
**F-14370 Moult (FR)**
Inventeur : **Boulouard, Michel**
**5 bd du Général Vanier**
**F-14000 Caen (FR)**
Inventeur : **Foloppe, Marie Paule**
**Neuville sur Tonques**
**F-61120 Vimoutiers (FR)**
Inventeur : **Robba, Max**
**4 rue Massillon**
**F-75004 Paris (FR)**
Inventeur : **Guardiola, Béatrice**
**6 rue Edouard Nortier**
**F-92200 Neuilly sur Seine (FR)**
Inventeur : **Devissaguet, Michelle**
**14 boulevard d'Inkermann**
**F-92200 Neuilly sur Seine (FR)**

(54) **Nouvelles pyrrolo[1,2-a]thiéno[3,2-f]diazépines[1,4], leur procédé de préparation et les compositions pharmaceutiques qui les contiennent.**

(57) L'invention concerne les dérivés de formule générale (I)

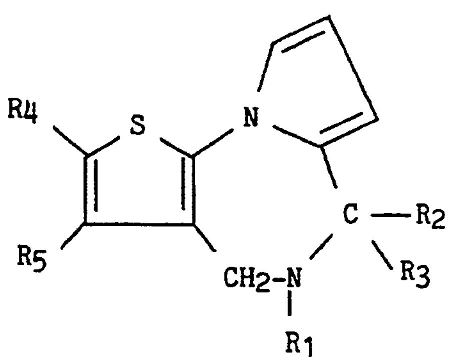

(I)

dans laquelle $R_1$, $R_2$, $R_3$, $R_4$ et $R_5$ sont tels que définis dans la description.

— leurs isomères, diastéroisomères, énantiomères ainsi que leurs sels d'addition à un acide minéral ou organique pharmaceutiquement acceptable.

— Médicament.

EP 0 446 140 A1

## NOUVELLES PYRROLO [1,2-a] THIENO [3,2-f] DIAZEPINES [1,4] LEUR PROCEDE DE PREPARATION ET LES COMPOSITIONS PHARMACEUTIQUES QUI LES CONTIENNENT

La présente invention concerne de nouvelles pyrrolo [1,2-a] thiéno [3,2-f] diazépines [1,4] , leur procedé de préparation et les compositions pharmaceutiques qui les contiennent.

Certaines aryle thiéno pyrrolo diazépines sont déjà connues pour leur action sur le système nerveux central(Japan Patent 77 100-496).

On connait également quelques aryle-6 dihydro-5,6 4H pyrrolo [1,2-a] thiéno [3,2-f] diazépines [1,4] dont la synthèse a été publiée, sans qu'une activité pharmacologique ne soit décrite (S. RAULT compte rendus Hebd. des séances de l'Académie des sciences, série C Vol 285 n° 11 31 octobre 1977 Paris pages 381-383).

Les pyrrolo [1,2-a] thiéno [3,2-f] diazépines [1,4] de la demanderesse sont des produits dotés de propriétés antihypoxiques remarquables qui sont très intéressantes dans le traitement du vieillissement cérébral et des accidents vasculaires cérébraux.

Le cerveau vieillissant puise en permanence dans ses réserves métaboliques pour maintenir en condition viable son métabolisme basal, toute demande fonctionnelle supplémentaire se traduisant par une insuffisance de réponse. Les produits de la demanderesse apportent une solution à ces problèmes en permettant l'augmentation de la consommation efficace d'oxygène quand il y a demande métabolique accrue sans la forcer à l'état basal. Leurs propriétés antihypermétaboliques les rendent également précieux dans le traitement des accidents vasculaires cérébraux.

Les produits de la demanderesse sont également extrêmement intéressants de par leurs effets métaboliques puisqu'ils possèdent d'importantes propriétés hypocholestérolémiantes, hypotriglycéridemiantes et hypoglycémiantes.

Enfin, ils possèdent à des degrés plus ou moins importants selon les structures, des propriétés antihypertensives, anxiolytiques et antagonistes du PAF (Platelet Antagonist Factor)

Plus spécifiquement, l'invention concerne les pyrrolo [1,2-a] thiéno [3,2-f] diazépines [1,4] de formule générale **(I)** :

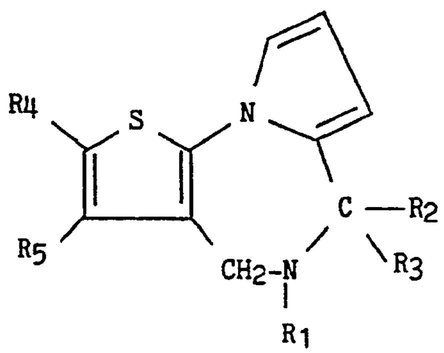

(I)

dans laquelle

– $R_1$ représente un atome d'hydrogène, un groupement alkyle inférieur de 1 à 6 atomes de carbone ramifié ou non éventuellement substitué par des groupements hydroxy, alkoxy, cycloalkyle, aryle, un groupement alkylsulfonyle ou arylsulfonyle éventuellement substitué sur le cycle aromatique par un ou plusieurs groupements alkyles inférieurs ou des halogènes,

– $R_4$ et $R_5$ représentent chacun indépendamment l'un de l'autre un atome d'hydrogène, un groupement alkyle inférieur de 1 à 6 atomes de carbone ramifié ou non éventuellement substitué par des groupements hydroxy, alkoxy, cycloalkyle, aryle substitué ou non, un groupement aryle ou aralkyle éventuellement substitué (le terme substitué associé aux expressions aryle ou aralkyle signifie que les noyaux aromatiques peuvent être substitués par un ou des groupements alkyle, nitro, alkoxy, trifluorométhyle ou halogène),

– $R_2$ et $R_3$ représentent chacun indépendamment l'un de l'autre un atome d'hydrogène, un groupement aryle ou aralkyle éventuellement substitué, un groupement alkyle inférieur de 1 à 6 atomes de carbone ramifié ou non éventuellement substitué par un ou des groupements hydroxy, alkoxy, oxo, cycloalkyle, aryle substitué ou non , ou un groupement -NR'R" pour lequel R' et R" représentent chacun indépendamment l'un de l'autre un atome d'hydrogène, un groupement - CO-R'"(R'" représente soit un phényle éventuellement substitué, soit un indolyle, soit un groupement anilino éventuellement substitué), un groupement alkyle inférieur, où forment ensemble avec l'atome d'azote qui les portent un système hétérocyclique mono

ou bicyclique comportant ou non un autre hétéroatome et éventuellement substitué par un groupement alkyl inférieur, aryle ou aralkyle éventuellement substitués, (le terme substitué pour les expressions aryle, phényle, anilino et aralkyle signifie que les noyaux aromatiques peuvent être substitués par un ou plusieurs groupements alkyle, nitro, alkoxy, trifluorométhyle ou halogène),

avec la réserve que, lorsque $R_1 = R_4 = R_5 = H$ et l'un des deux substituants $R_2$ ou $R_3$ est un atome d'hydrogène, alors l'autre substituant ne peut représenter ni un atome d'hydrogène, ni un groupement méthoxy, ni un groupement aryle éventuellement substitué,

– leurs isomères, diastéroisomères, énantiomères,

– leurs sels d'addition à un acide minéral ou organique, pharmaceutiquement acceptable.

L'invention s'étend également aux procédés d'obtention des composés de formule générale **(I)**. Les procédés optima d'obtention de ces composés varient selon la nature des substituants $R_1$, $R_2$ $R_3$, $R_4$ et $R_5$ et sont caractérisés en ce que :

- soit l'on part d'un dérivé de formule générale **(II)** :

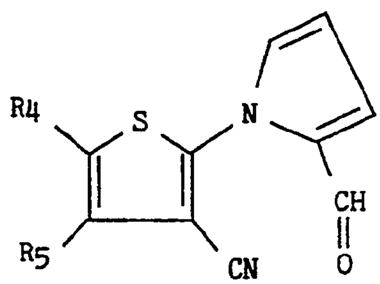

(II)

dans laquelle $R_4$ et $R_5$ ont la même signification que dans le dérivé de formule générale **(I)** que l'on fait réagir dans un mélange d'une solution aqueuse d'hydroxyde de sodium, d'éthanol et de peroxyde d'hydrogène avec une méthyle cétone de formule générale **(III)** :

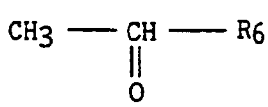

(III)

dans laquelle $R_6$ est un groupement cycloalkyle, alkyle inférieur de 1 à 6 atomes de carbone ramifié ou non éventuellement substitué par des groupements alkoxy, cycloalkyle, aryle, de manière à obtenir la dihydro-5,6 oxo-4 4H pyrrolo [1,2-a] thiéno [3,2-f] diazépine [1,4] de formule générale **(IV)** :

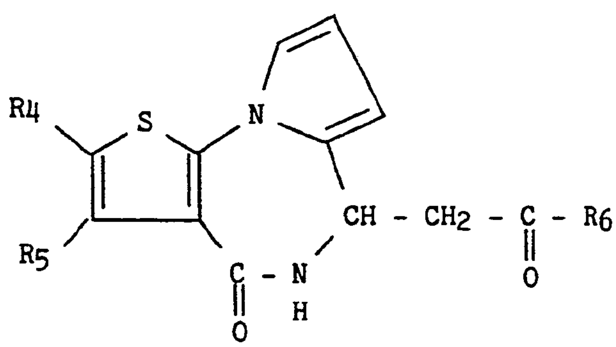

(IV)

dans laquelle $R_6$ a la même signification que dans la formule générale **(III)** et $R_4$ et $R_5$ la même signification que dans le dérivé de formule générale **(I)** que l'on réduit en milieu aprotique par un hydrure métallique mixte de manière à obtenir la dihydro-5,6 4H pyrrolo [1,2-a] thiéno [3,2-f] diazépine [1,4] de formule générale **(V)** :

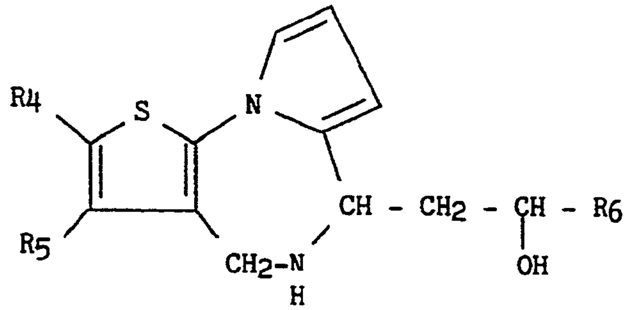

(V)

dans laquelle $R_4$, $R_5$ et $R_6$ ont la même signification que dans la formule **(III)** que l'on peut traiter en solution éthérée par de l'acide chlorhydrique gazeux pour obtenir le chlorure de dihydro-5,6 -4H -5H$^{\oplus}$ pyrrolo [1,2-a] thiéno [3,2-f] diazépinium [1,4] de formule générale **(VI)** :

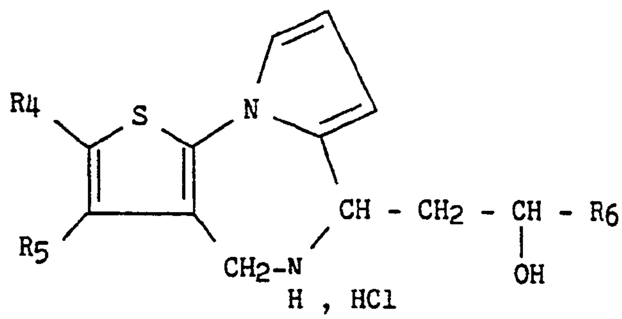

(VI)

dans laquelle $R_4$, $R_5$ et $R_6$ ont la même signification que la formule **(III)**,

- soit l'on part du dérivé de formule générale **(VII)** :

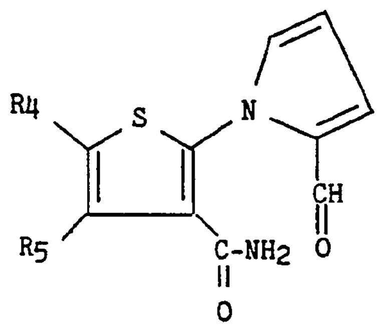

(VII)

dans laquelle $R_4$ et $R_5$ ont la même signification que dans le dérivé de formule générale **(I)** que l'on fait réagir en suspension dans l'eau avec une amine tertiaire de manière à obtenir le dérivé de formule générale **(VIII)** :

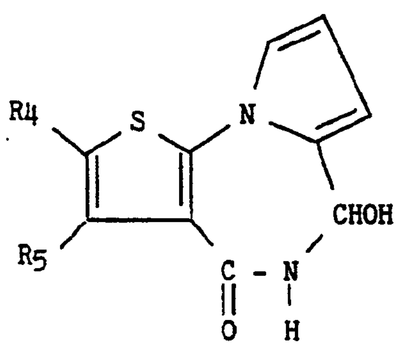

(VIII)

dans laquelle $R_4$ et $R_5$ ont la même signification que dans le dérivé de formule générale **(I)** que l'on réduit en milieu aprotique par un hydrure métallique mixte de manière à obtenir le dérivé de formule générale **(IX)** :

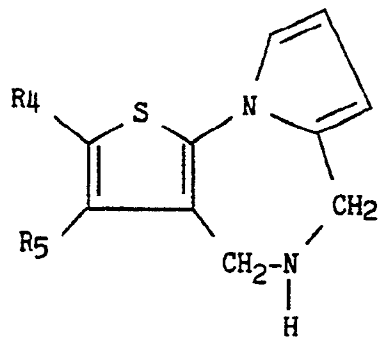

**(IX)**

dans laquelle $R_4$ et $R_5$ ont la même signification que dans le dérivé de formule générale **(I)** que l'on fait réagir en présence d'amine avec un dérivé halogéné de formule générale **(X)** :

$R_1Cl$ **(X)**

dans laquelle $R_1$ a la même signification que dans le dérivé de formule générale **(I)** de manière à obtenir le dérivé de formule générale **(XI)** :

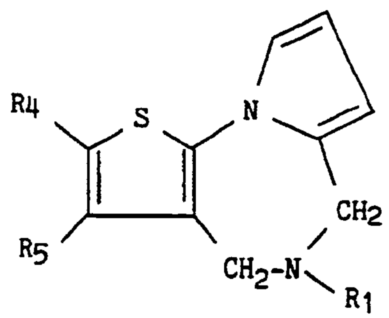

**(XI)**

dans laquelle $R_1$, $R_4$ et $R_5$ ont la même signification que dans le dérivé de formule générale **(I)**,

- soit l'on part du dérivé de formule générale **(XII)** :

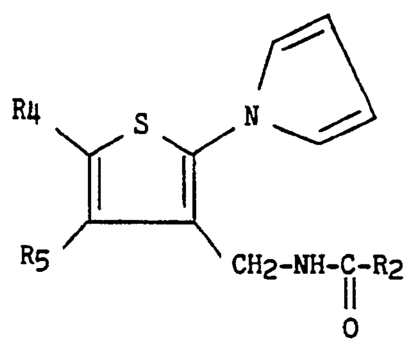

**(XII)**

dans laquelle $R_2$ ,$R_4$ et $R_5$ ont la même signification que dans le dérivé de formule générale **(I)** que l'on cyclise avec de l'oxychlorure de phosphore de manière à obtenir le dérivé de formule générale **(XIII)** :

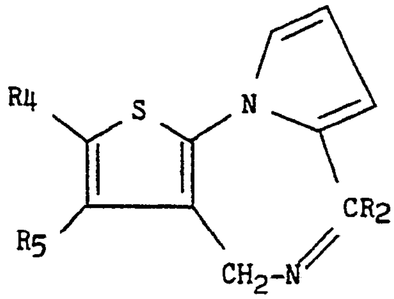

(XIII)

dans laquelle $R_2$, $R_4$ et $R_5$ ont la même signification que dans le dérivé de formule générale **(I)** que l'on réduit en milieu aprotique par un hydrure métallique mixte de manière à obtenir la dihydro-5,6 4H pyrrolo [1,2-a] thiéno [3,2-f] diazépine [1,4] de formule générale **(XIV)** :

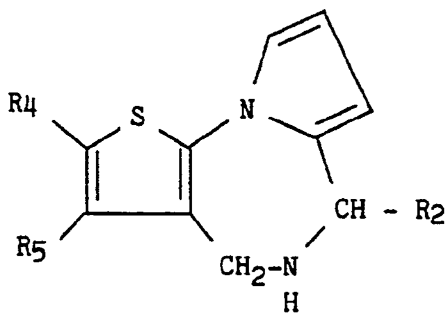

(XIV)

dans laquelle $R_2$, $R_4$ et $R_5$ ont la même signification que dans le dérivé de formule générale **(I)** que l'on peut traiter en solution éthérée par de l'acide chlorhydrique gazeux pour obtenir le chlorure de dihydro-5,6 4H $5H^{\oplus}$ pyrrolo [1,2-a] thiéno [3,2-f] diazepinium [1,4] de formule générale **(XV)**:

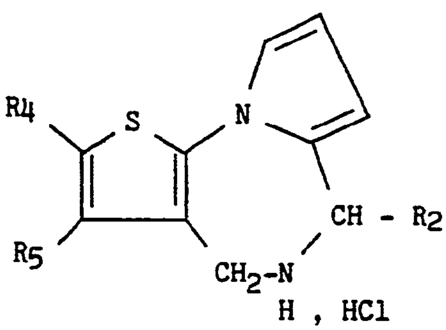

(XV)

dans laquelle $R_2$, $R_4$ et $R_5$ ont la même signification que dans le dérivé de formule générale **(I)**,

- soit l'on part du dérivé de formule générale **(XVI)** :

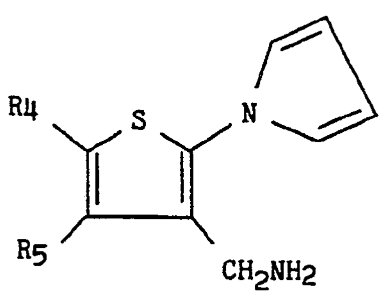

(XVI)

dans laquelle $R_4$ et $R_5$ ont la même signification que dans le dérivé de formule générale **(I)** que l'on fait réagir dans de l'alcool au reflux avec un aldéhyde de formule générale **(XVII)** :

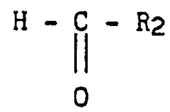

(XVII)

dans laquelle $R_2$ a la même signification que dans le dérivé de formule générale **(I)** de manière à obtenir le composé de formule générale **(XVIII)** :

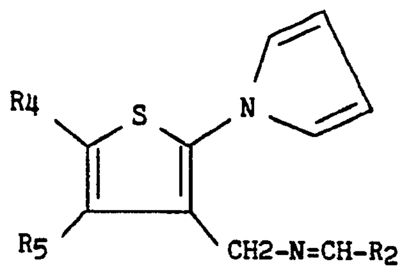

(XVIII)

dans laquelle $R_2$,$R_4$ et $R_5$ ont la même signification que dans le dérivé de formule générale **(I)** que l'on traite en solution éthérée par de l'acide chlorhydrique gazeux de manière à obtenir le chlorure de dihydro-5,6 4H 5H⊕ pyrrolo [1,2-a] thiéno [3,2-f] diazépinium [1,4] de formule générale **(XV)** dans laquelle $R_2$, $R_4$ et $R_5$ ont la même signification que dans le dérivé de formule générale **(I)**,

- soit l'on part du dérivé de formule générale **(XIX)** :

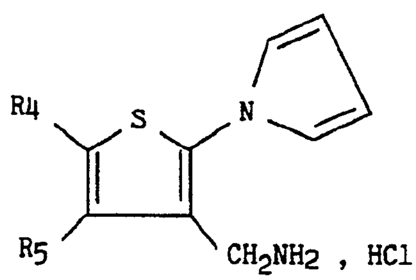

(XIX)

dans laquelle $R_4$ et $R_5$ ont la même signification que dans le dérivé de formule générale **(I)** que l'on fait réagir au reflux dans de l'alcool et en présence d'acide chlorhydrique concentré avec une cétone de formule générale **(XX)** :

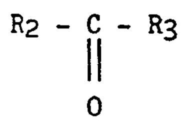

(XX)

dans laquelle $R_2$ et $R_3$ ont la même signification que dans le dérivé de formule générale **(I)** de manière à obtenir le chlorure de dihydro-5,6 4H 5H⊕ pyrrolo [1,2-a] thiéno [3,2-f] diazépinium [1,4] de formule générale **(XXI)** :

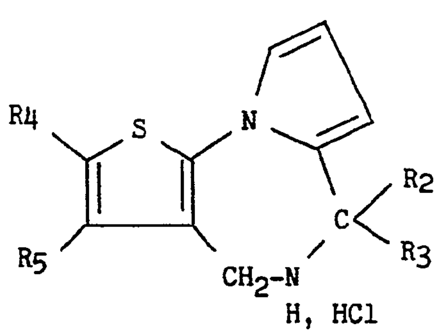

(XXI)

dans laquelle $R_2$, $R_3$, $R_4$ et $R_5$ ont la même signification que dans le dérivé de formule générale **(I)**,

- soit l'on part du dérivé de formule générale **(XIX)** dans laquelle $R_4$ et $R_5$ ont la même signification que dans le dérivé de formule générale **(I)** que l'on fait réagir en milieu alcoolique avec de l'éthoxy-2 hydroxy-2 acétate d'éthyle de manière à obtenir le dérivé de formule générale **(XXII)** :

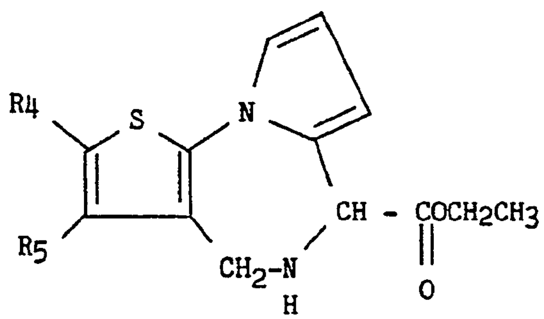

**(XXII)**

dans laquelle $R_4$ et $R_5$ ont la même signification que dans le dérivé de formule générale **(I)** que l'on réduit en solution alcoolique par un hydrure métallique mixte de manière à obtenir l'hydroxyméthyl-6 dihydro-5,6 4H pyrrolo [1,2-a] thiéno [3,2-f] diazépine [1,4] de formule générale **(XXIII)** :

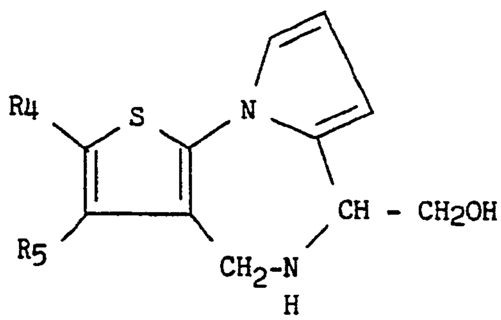

**(XXIII)**

dans laquelle $R_4$ et $R_5$ ont la même signification que dans le dérivé de formule générale **(I)**,

- soit l'on part du dérivé de formule générale **(VIII)** dans laquelle $R_4$ et $R_5$ ont la même signification que dans le dérivé de formule générale **(I)** que l'on fait réagir au reflux de l'acétonitrile avec une amine de formule générale **(XXIV)**:

HNR'R" **(XXIV)**

dans laquelle R'et R" ont la même signification que dans le dérivé de formule générale **(I)** de manière à obtenir le dérivé de formule générale **(XXV)**:

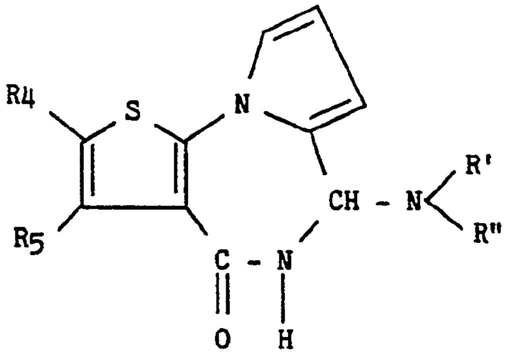

**(XXV)**

dans laquelle $R_4$, $R_5$, R' et R" ont la même signification que dans le dérivé de formule générale **(I)** que l'on réduit en milieu aprotique par un hydrure métallique mixte de manière à obtenir le dérivé de formule générale **(XXVI)**:

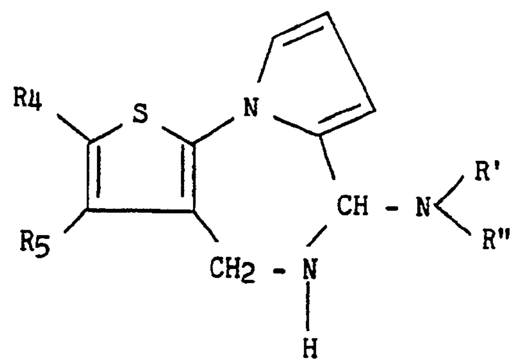

(XXVI)

dans laquelle $R_4$, $R_5$, R′ et R″ ont la même signification que dans le dérivé de formule générale **(I)**.

Les composés de formule générale **V**, **VI**,**IX**, **XI**, **XIV**, **XV**, **XXI**, **XXII** , **XXIII** et **XXVI** font partie de l'invention et représentent les composés de formule générale **(I)**.

Les pyrrolo [1,2-a] thiéno [3,2-f] diazépines [1,4] de formule générale **(I)** ainsi que leurs sels d'addition à un acide minéral ou organique pharmaceutiquement acceptable sont des principes actifs possédant de remarquables propriétés pharmacologiques et thérapeutiques.

Ces composés possèdent de fortes propriétés antihypoxiques qui les rendent précieux dans le traitement, entre autres, du vieillissement cérébral, des accidents vasculaires cérébraux et plus généralement, des syndromes ischémiques de toute localisation. Ils sont capables, chez la souris, de prolonger la survie du tissu cérébral lors d'hypoxie aigue.

Ces composés présentent également de très intéressantes propriétés métaboliques puis qu'ils sont fortement hypocholestérolémiants, hypotriglycéridémiants, hypolipidémiants et hypoglycémiants d'où de très nombreuses applications thérapeutiques potentielles (obésité, athérome, hyperlipidémie, hypercholestérolémie...)

Ces composés possèdent enfin, et à des degrés plus ou moins importants selon leur structure, des propriétés antihypertensives, anxiolytiques et antagonistes du PAF.

Les pyrrolo [1,2-a] thiéno [3,2-f] diazépines [1,4] de formule générale **(I)** ainsi que leurs sels d'addition à un acide minéral ou organique pharmaceutiquement acceptables comme par exemple les acides chlorhydrique, méthanesulfonique, citrique, maléique peuvent être élaborés en préparations pharmaceutiques selon des procédés généralement connus comme par exemple en comprimés, gelules, dragées, solutions buvables, solutions injectables, suspensions buvables, émulsions, suppositoires.

Outre les excipients inertes, non toxiques et pharmaceutiquement acceptables tels par exemple l'eau distillée, le glucose, le lactose, l'amidon, le talc, les huiles végétales, l'éthylène glycol etc., ces préparations peuvent également contenir des agents de préservation, stabilisants, mouillants émulsifiants etc..

Les compositions ainsi obtenues se présentent généralement sous forme dosée et peuvent contenir selon les affections traitées, l'âge et le sexe du malade de 0,1 à 500 mg de principe actif. Elles peuvent selon le cas être administrées par voie orale, rectale ou parentérale à la dose de 0,1 à 500 mg de une à plusieurs fois par jour.

Les exemples suivants illustrent l'invention et ne la limitent en aucune façon.

Les caractéristiques de spectrométrie $RMN^1$ H sont regroupés dans les tableaux I.

**EXEMPLE 1 : Chlorure d'(hydroxy-2 pentyl)-6 dihydro-5,6 4H 5H$^{\oplus}$ Pyrrolo [1,2-a] thiéno [3,2-f] diazépinium [1,4]**

**Stade I** : (oxo-2 pentyl-1)-6 dihydro-5,6 oxo-4 4H pyrrolo [1,2-a] thiéno [3,2-f] diazépine [1,4]

Une solution de 12 g (0,059 moles) de (formyl-2 pyrrolyl-1)-2 cyano-3 thiophène dans un mélange de 200 $cm^3$ de pentanone-2, 200 $cm^3$ d'éthanol, 200 $cm^3$ d'hydroxyde de sodium 5N et 15 $cm^3$ de peroxyde d'hydrogène à 33 % est chauffée au reflux pendant une heure. L'éthanol et la pentanone-2 sont ensuite éliminés sous pression réduite et le reste du milieu réactionnel versé sur 200 $cm^3$ d'eau froide. Le précipité formé est isolé par filtration, lavé à l'eau, séché puis recristallisé dans un mélange éther - acétone.

On obtient ainsi 11,5 g (68 %) d'(oxo-2 pentyl-1)-6 dihydro-5,6 oxo-4 4H pyrrolo[1,2-a] thiéno [3,2-f] diazépine [1,4] sous forme de cristaux jaunes.

F = 186°c (éther - acétone)

Microanalyse $C_{15}H_{16}N_2O_2S$ M = 288,36

| | C | H | N | S |
|---|---|---|---|---|
| Calculé | C 62,49 % | H 5,59 % | N 9,72 % | S 11,10 % |
| Trouvé | C 62,48 % | H 5,50 % | N 9,72 % | S 11,23 % |

Spectre IR (KBr)

| | |
|---|---|
| 3275 et 3170 $cm^{-1}$ | bandes NH |
| 3050, 2950, 2920 et 2870 $cm^{-1}$ | bandes CH |
| 1705 $cm^{-1}$ | bande C = 0 (cétone) |
| 1640 $cm^{-1}$ | bande C = 0 (lactame) |

**Stade II** : Chlorure d'(hydroxy-2 pentyl)-6 dihydro-5,6 4H 5H⊕ pyrrolo [1,2-a] thiéno [3,2-f] diazépinium [1,4].

Une solution de 2,6 g (0,009 moles) d'(oxo-2 pentyl-1)-6 dihydro-5,6 oxo-4 4H pyrrolo[1,2-a] thiéno [3,2-f] diazépine [1,4] dans 550 $cm^3$ de dichlorométhane est additionnée goutte à goutte à une suspension de 1,50 g (0,04 moles) d'hydrure de lithium et d'aluminium dans 20 $cm^3$ d'éther éthylique. Le mélange réactionnel est agité à température ambiante pendant 30 minutes puis chauffé au reflux pendant 6 heures. Après refroidissement, la solution obtenue est jetée sur 250 g de glace. L'émulsion formée est filtrée, la phase organique décantée et la phase aqueuse extraite avec 200 $cm^3$ de chlorure de méthylène. Les phases organiques sont rassemblées, séchées sur du chlorure de calcium, purifiées sur noir animal puis concentrées sous pression réduite. L'huile résiduelle est reprise en solution dans 300 $cm^3$ d'éther éthylique et le chlorydrate précipité par barbottage d'acide chlorhydrique gazeux. Le précipité obtenu est filtré, lavé à l'éther et recristallisé dans l'isopropanol.

On obtient ainsi 1,4 g (50 %) de chlorure d'(hydroxy-2 pentyl)-6 dihydro-5,6 4H 5H⊕ pyrrolo [1,2-a] thiéno [3,2-f] diazépinium [1,4] sous forme de cristaux gris.

F = 214°c (isopropanol)

Microanalyse

Microanalyse $C_{15}H_{21}N_2OS\ Cl$ M = 312,86

| | C | H | S | Cl |
|---|---|---|---|---|
| Calculé | C 57,59 % | H 6,76 % | S 10,25 % | Cl 11,33 % |
| Trouvé | C 57,80 % | H 7,09 % | S 9,59 % | Cl 10,69 % |

Spectre IR (KBr)

| | |
|---|---|
| 3350 $cm^{-1}$ | bande OH |
| 3310 $cm^{-1}$ | bande CH |
| 2960 à 2550 $cm^{-1}$ | bande large $NH_2^{\oplus}Cl^-$ |

1565, 1430, 1330, 1130, 1100, 960 et 735 $cm^{-1}$ bandes principales.

**EXEMPLE 2 : Chlorure de (cyclopropyl-2 hydroxy-2 éthyl)-6 dihydro-5,6 4H 5H⊕ pyrrolo [1,2-a] thiéno [3,2-f] diazépinium [1,4].**

**Stade I** : (cyclopropyl-2 oxo-2 éthyl)-6 dihydro -5,6 oxo-4 4H pyrrolo [1,2-a] thiéno [3,2-f] diazépine [1,4]

Même protocole opératoire que pour le stade I de l'exemple 1 en partant de 6 g (0,029 moles) de (formyl-2 pyrrolyl-1)-2 cyano-3 thiophène et 50 $cm^3$ de cyclopropyl méthyl cétone.

On obtient ainsi 4,80 g (57 %) de (cyclopropyl-2 oxo-2 éthyl)-6 dihydro-5,6 oxo-4 4H pyrrolo [1,2-a] thiéno

[3,2-f] diazépine [1,4] sous forme de cristaux blancs.
F = 200° c (éther éthylique - acétone)

| | | |
|---|---|---|
| Microanalyse | $C_{15}H_{14}N_2O_2S$ M = 286,35 | |
| | Calculé | C 62,91 % H 4,92 % N 9,78 % S 11,19 % |
| | Trouvé | C 62,87 % H 4,93 % N 9,81 % S 11,05 % |

| | | |
|---|---|---|
| Spectre IR (KBr) | 3270 et 3180 $cm^{-1}$ | bandes NH |
| | 3120, 3090, 3050 et 2890 $cm^{-1}$ | bandes CH |
| | 1685 et 1640 $cm^{-1}$ | bandes C = 0 |

**Stade II** : Chlorure de (cyclopropyl-2 hydroxy-2 éthyl)-6 dihydro-5,6 4H 5H$^{\oplus}$ pyrrolo [1,2-a] thiéno [3,2-f] diazépinium [1,4].

Même protocole opératoire que pour le stade II de l'exemple 1 en partant de 1,2 g (0,0042 moles) de (cyclopropyl-2 oxo-2 éthyl)-6 dihydro-5,6 oxo-4 4H pyrrolo [1,2-a] thiéno [3,2-f] diazépine [1,4] dans 400 $cm^3$ de dichlorométhane et 0,7 g (0,017 moles) d'hydrure de lithium et d'aluminium dans 20 $cm^3$ d'éther éthylique.

On obtient ainsi 0,7 g (54 %) de chlorure de (cyclopropyl-2 hydroxy-2 éthyl)-6 dihydro-5,6 4H 5H$^{\oplus}$ pyrrolo [1,2-a] thiéno [3,2-f] diazépinium [1,4] sous forme de cristaux blancs.
F = 236°c (isopropanol)

| | | |
|---|---|---|
| Microanalyse | $C_{15}H_{19}$ $N_2OS$ Cl M = 310,84 | |
| | Calculé | C 57,96 % H 6,16 % N 9,01 % Cl 11,41 % |
| | Trouvé | C 57,74 % H 6,07 % N 8,80 % Cl 11,62 % |

| | | |
|---|---|---|
| Spectre IR (KBr) | 3350 $cm^{-1}$ | bande OH |
| | 3310, 2980 $cm^{-1}$ | bande CH |
| | 2970 à 2540 $cm^{-1}$ | bande large $NH_2^{\oplus}Cl^-$ |
| | 1565, 1480, 1330, 1100, 1025, 960, 825, 760 et 735 cm-1 bandes principales. | |

**EXEMPLE 3 : Chlorure d'(hydroxy-2 méthyl-3 butyl)-6 dihydro-5,6 4H 5H$^{\oplus}$ pyrrolo [1,2-a] thiéno [3,2-f] diazépinium [1,4].**

**Stade I** : (méthyl-3 oxo-2 butyl-1)-6 dihydro-5,6 oxo-4 4H pyrrolo [1,2-a] thiéno [3,2-f] diazépine [1,4].

Même protocole opératoire que pour le stade I de l'exemple 1 en partant de 12 g (0,059 moles) de (formyl-2 pyrrolyl-1)-2 cyano-3 thiophène et 100 $cm^3$ d'isopropyl méthyl cétone.

On obtient ainsi 11,1 g (65 %) de (méthyl-3 oxo-2 butyl-1)-6 dihydro-5,6 oxo-4 4H pyrrolo [1,2-a] thiéno [3,2-f] diazépine [1,4] sous forme de cristaux jaunes
F : 160° c (éthanol)

Microanalyse $C_{15}H_{16}N_2O_2S$ M = 288,35
Calculé C 62,49 % H 5,59 % N 9,72 % S 11,10 %
Trouvé C 62,50 % H 5,80 % N 9,69 % S 11,07 %

| | | |
|---|---|---|
| Spectre IR (KBr) | 3280 et 3175 $cm^{-1}$ | bandes NH |
| | 3060, 2960, 2930 et 2880 $cm^{-1}$ | bandes CH |
| | 1710 et 1650 $cm^{-1}$ | bandes C = O |

**Stade II** : Chlorure d'(hydroxy-2 méthyl-3 butyl)-6 dihydro-5,6 4H 5H$^{\oplus}$ pyrrolo [1,2-a] thiéno [3,2-f] diazépinium [1,4]

Même protocole opératoire que pour le stade II de l'exemple 1 en partant de 1,4 g (0,0049 moles) de (méthyl-3 oxo-2 butyl)-6 dihydro-5,6 oxo-4 4H pyrrolo [1,2-a] thiéno [3,2-f] diazépine [1,4] dans 400 $cm^3$ de dichlorométhane et 0,76 g (0,020 moles) d'hydrure de lithium et d'aluminium dans 20 $cm^3$ d'éther éthylique.

On obtient ainsi 0,8 g (52 %) de chlorure d'(hydroxy-2 méthyl-3 butyl)-6 dihydro-5,6 4H 5H$^{\oplus}$ pyrrolo [1,2-a] thiéno [3,2-f] diazépinium [1,4] sous forme de poudre blanche.

F = 236°c (isopropanol)

Microanalyse $C_{15}H_{21}N_2OSCl$ M = 312,86
Calculé C 57,59 % H 6,76 % S 10,25 % Cl 11,33 %
Trouvé C 57,50 % H 6,88 % S 9,67 % Cl 11,02 %

| | | |
|---|---|---|
| Spectre IR (KBr) | 3380 $cm^{-1}$ | bande OH |
| | 3110 $cm^{-1}$ | bande CH |
| | 2960 à 2560 $cm^{-1}$ | bande large $NH_2^{\oplus}Cl^-$ |
| | 1570, 1480, 1330, 1260, 1090 et 715 $cm^{-1}$ bandes principales. | |

**EXEMPLE 4 : Chlorure de (phényl-2 hydroxy-2 éthyl)-6 dihydro-5,6 4H 5H$^{\oplus}$ pyrrolo [1,2-a] thiéno [3,2-f] diazépinium [1,4].**

**Stade I:** Phénacyl-6 dihydro-5,6 oxo-4 4H pyrrolo [1,2-a] thiéno [3,2-f] diazépine [1,4]

Même protocole opératoire que pour le stade I de l'exemple 1 en partant de 1 g (0,0049 moles) de (formyl-2 pyrrolyl-1)-2 cyano-3 thiophène 20 $cm^3$ d'acétophénone, 100 $cm^3$ d'hydroxyde de sodium 6N, 50 $cm^3$ d'éthanol et 5 $cm^3$ de peroxyde d'hydrogène à 33 %. Après élimination de l'éthanol sous pression réduite, le milieu réactionnel est versé sur 100 $cm^3$ d'eau. Le précipité huileux qui est formé d'acétophénone et de l'oxodiazépine est extrait avec de l'éther éthylique.La phase éthérée est lavée à l'eau, séchée sur sulfate de magnésium et concentrée sous pression réduite. L'acétophénone est éliminée par lavage du résidu par un mélange ether de pétrole - éther éthylique (50 - 50). Le solide insoluble restant est filtré, séché et recristallisé dans l'éthanol.

On obtient ainsi 0,7 g (44 %) de phénacyl-6 dihydro-5,6 oxo-4 4H pyrrolo [1,2-a] thiéno [3,2-f] diazépine [1,4] sous forme de cristaux blancs.

F = 205°c (éthanol)

Microanalyse $C_{18}H_{14}N_2O_2S$ M = 322,37

Calculé C 67,07 % H 4,38 % N 8,69 % S 9,93 %

Trouvé C 66,83 % H 4,63 % N 8,80 % S 9,68 %

Spectre IR (KBr) 3320 et 3260 $cm^{-1}$ bandes NH

3090, 3060 et 2920 $cm^{-1}$ bandes CH

1675 et 1640 $cm^{-1}$ bandes C = O

1595, 1535, 1480, 1430, 1325, 1215, 755 et 685 $cm^{-1}$ bandes principales.

**Stade II** : Chlorure de (phényl-2 hydroxy-2 éthyl)-6 dihydro-5,6 4H 5H$^{\oplus}$ pyrrolo [1,2-a] thiéno [3,2-f] diazépinium [1,4]

Même protocole opératoire que pour le stade II de l'exemple 1 en partant de 1,6 g (0,005 moles) de phénacyl-6 dihydro-5,6 oxo-4 pyrrolo [1,2-a] thiéno [3,2-f] diazépine [1,4] dans 450 cm³ de dichlorométhane et 0,76 g (0,02 moles)d'hydrure de lithium et d'aluminium dans 20 cm³ d'éther éthylique.

On obtient ainsi 0,9 g (52 %) de chlorure de (phényl-2 hydroxy-2 ethyl)-6 dihydro-5,6 4H 5H$^{\oplus}$ pyrrolo [1,2-a] thiéno [3,2-f] diazépinium [1,4] sous forme de cristaux blancs.

F = 186°c (isopropanol)

Microanalyse $C_{18}H_{19}N_2OSCl$ M = 346,87

Calculé C 62,33 % H 5,52 % S 9,24 % Cl 10,22 %

Trouvé C 61,96 % H 5,77 % S 8,89 % Cl 9,89 %

Spectre IR (KBr) 3340 $cm^{-1}$ bandes OH

3110 $cm^{-1}$ bande CH

2970 à 2540 $cm^{-1}$ bande large $NH_2^{\oplus}Cl^-$

1565, 1330, 1100, 1070, 960, 865, 735 et 705 $cm^{-1}$ bandes principales.

**EXEMPLE 5 : Chlorure d'(hydroxy-2 propyl)-6 dihydro-5,6 4H 5H$^{\oplus}$ pyrrolo [1,2-a] thiéno [3,2-f] diazépinium [1,4] .**

**Stade I** : (oxo-2 propyl)-6 dihydro-5,6 oxo-4 4H pyrrolo [1,2-a] thiéno [3,2-f] diazépine.[1,4]

Même protocole opératoire que pour le stade I de l'exemple 1 à partir d'acétone.

**Stade II** : Chlorure d'(hydroxy-2 propyl)-6 dihydro-5,6 4H 5H$^{\oplus}$ pyrrolo [1,2-a] thiéno [3,2-f] diazépinium [1,4].

Même protocole opératoire que pour le stade II de l'exemple 1 en partant de 5 g (0,019 moles) d'(oxo-2 propyl)-6 dihydro-5,6 oxo-4 4H pyrrolo [1,2-a] thiéno [3,2-f] diazépine [1,4] dans 500 cm³ de dichlorométhane et 2,9 g (0,076 moles) d'hydrure de lithium et d'aluminium dans 20 cm³ d'éther éthylique.

On obtient ainsi 2,2 g (41 %) de chlorure d'(hydroxy-2 propyl)-6 dihydro-5,6 4H 5H$^{\oplus}$ pyrrolo [1,2-a] thiéno [3,2-f] diazépinium [1,4] sous forme de cristaux beiges.

F = 226°c (isopropanol)

Microanalyse $C_{13}H_{17}N_2OSCl$ M = 284,8

Calculé C 54,82 % H 6,02 % S 11,26 % Cl 12,45 %

Trouvé C 54,98 % H 6,04 % S 11,09 % Cl 12,67 %

Spectre IR (KBr) 3390 $cm^{-1}$ bande OH

3110 $cm^{-1}$ bande CH

2960 à 2580 $cm^{-1}$ bande large $NH_2^{\oplus}Cl^-$

1570, 1480, 1330, 1180, 1100, 730 et 700 $cm^{-1}$ bandes principales.

**EXEMPLE 6 : (Hydroxy-2 propyl)-6 dihydro-5,6 4H pyrrolo [1,2-a] thiéno [3,2-f] diazépine [1,4].**

Une solution de 1 g (0,0035 moles) de chlorure d' (hydroxy-2 propyl)-6 dihydro-5,6 4H 5H$^{\oplus}$ pyrrolo [1,2-a] thiéno [3,2-f] diazépinium [1,4] dans 50 $cm^3$ d'eau est alcalinisée à l'aide de pastilles d'hydroxyde de sodium jusqu'à PH 14. L'émulsion obtenue est extraite par 3 fois 50 $cm^3$ d'éther éthylique. Les phases organiques sont décantées, rassemblées, séchées sur sulfate de magnésium et concentrées sous pression réduite jusqu'à obtention d'un produit brut qui est recristallisé dans l'éther éthylique.

On obtient ainsi 0,70 g (80 %) d'(hydroxy-2 propyl)-6 dihydro-5,6, 4H pyrrolo [1,2-a] thiéno [3,2-f] diazépine [1,4] sous forme de cristaux blancs.

F = 130°c ($Et_2O$)

Microanalyse $C_{13}H_{16}N_2OS$ M = 248,34

Calculé C 62,87 % H 6,49 % N 11,28 % S 12,91 %

Trouvé C 62,74 % H 6,55 % N 11,21 % S 12,75 %

Spectre IR (KBr) 3240 $cm^{-1}$ bande NH

3080, 2960, 2920 et 2830 $cm^{-1}$ bandes CH

1580, 1480, 1310, 1135 et 705 $cm^{-1}$ bandes principales.

**EXEMPLE 7 : (phényl-2 hydroxy-2 éthyl)-6 dihydro-5,6 4H pyrrolo [1,2-a] thiéno [3,2-f] diazépine [1,4].**

Même protocole opératoire que pour l'exemple 6 en partant de 1 g (0,0029 moles) de chlorure de (phényl-2 hydroxy-2 éthyl)-6 dihydro-5,6 4H 5H$^{\oplus}$ pyrrolo [1,2-a] thiéno [3,2-f] diazépinium [1,4].

On obtient ainsi 0,72 g (80 %) de (phényl-2 hydroxy-2 éthyl)-6 dihydro-5,6 4H pyrrolo [1,2-a] thiéno [3,2-f] diazépine [1,4] sous forme de cristaux blancs.

F = 190°c ($Et_2O$)

Microanalyse $C_{18}H_{16}N_2O_2S$ M = 324,40
Calculé C 66,65 % H 4,97 % N 8,64 % S 9,88 %
Trouvé C 66,56 % H 4,89 % N 8,52 % S 9,68 %

Spectre IR (KBr) 3300 $cm^{-1}$ bande NH
3100 et 2920 $cm^{-1}$ bandes CH
1580, 1480, 1330, 1095 et 705 $cm^{-1}$ bandes principales.

**EXEMPLE 8 : para Tolylsulfonyl-5 dihydro-5,6 4H pyrrolo [1,2-a] thiéno [3,2-f] diazépine [1,4].**

**Stade I** : hydroxy-6 dihydro-5,6 oxo-4 4H pyrrolo [1,2-a] thiéno [3,2-f] diazépine [1,4]

A une suspension de (formyl-2 pyrrolyl-1)-2 carboxamido-3 thiophène dans 60 $cm^3$ d'eau on additionne 0,5 $cm^3$ de triéthylamine. Le milieu réactionnel est agité 12 heures à température ambiante. Le produit passe progressivement en solution puis reprécipite. Le précipité obtenu est isolé par filtration, lavé à l'eau, isolé puis recristallisé dans l'eau.

On obtient ainsi 1,35 g (68 %) d'hydroxy-6 dihydro-5,6 oxo-4 4H pyrrolo [1,2-a] thiéno [3,2-f] diazépine [1,4] sous forme de cristaux beiges.

F = 166°c

Microanalyse $C_{16}H_8N_2O_2S$ M = 220,45
Calculé C 54,53 % H 3,66 % N 12,71 % S 14,56 %
Trouvé C 54,36 % H 3,69 % N 12,58 % S 14,39 %

Spectre IR (KBr) 3340 $cm^{-1}$ bande OH
3220 $cm^{-1}$ bande NH
3120 et 3110 $cm^{-1}$ bandes CH
1610 $cm^{-1}$ bande C = 0
1500, 1450, 1320, 1205, 1005, 830 et 720 $cm^{-1}$ bandes principales.

**Stade II** : Dihydro-5,6 4H pyrrolo [1,2-a] thiéno [3,2-f] diazépine [1,4]

Une solution de 3 g (0,014 moles) d'hydroxy-6 dihydro-5,6 oxo-4 4H pyrrolo [1,2-a] thiéno [3,2-f] diazépine [1,4] dans 300 $cm^3$ de dichlorométhane est additionnée goutte à goutte à une suspension de 2,08 g (0,055 moles) d'hydrure d'aluminium et de lithium dans 15 $cm^3$ d'éther éthylique. Le milieu réactionnel est agité 30 minutes à température ambiante puis chauffé au reflux pendant 6 heures.

Après refroidissement, la solution obtenue est jetée sur 200 g de glaoe et l'émulsion formée est essorée. La phase organique est décantée et la phase aqueuse extraite par 200 $cm^3$ de dichlorométhane. Les phases organiques sont rassemblées, séchées sur chlorure de calcium, purifiées sur noir animal et concentrées sous pression partielle. On obtient ainsi 2,4 g (90 %) de dihydro-5,6 4H pyrrolo [1,2-a] thiéno [3,2-f] diazépine [1,4] sous forme d'huile.

**Stade III** : para Tolylsulfonyl-5 dihydro-5,6 4H pyrrolo [1,2-a] thiéno [3,2-f] diazépine [1,4].

A une solution de 1 g (0,0053 moles) de dihydro-5,6 4H pyrrolo [1,2-a] thiéno [3,2-f] diazépine [1,4] dans 10 cm³ de pyridine, on ajoute un excès de chlorure de paratoluène sulfonyle puis le mélange réactionnel est agité à température ambiante pendant une heure. La pyridine est éliminée par distillation sous pression réduite et le résidu huileux trituré dans 100 cm³ d'eau. L'émulsion formée est extraite par 3 fois 100 cm³ d'éther éthylique.

Les phases organiques sont décantées, rassemblées, lavées à l'eau acidulée, séchées sur sulfate de magnésium et enfin concentrée sous pression réduite.

Le solide obtenu est ensuite recristallisé dans de l'éther éthylique.

On obtient ainsi 0,5 g (27%) de para tolylsulfonyl-5 dihydro-5,6 4H pyrrolo [1,2-a] thiéno [3,2-f] diazépine [1,4] sous forme de cristaux beiges.

F = 173°c (ether ethylique)

Microanalyse $C_{17}H_{16}N_2O_2S$ M = 344,44

Calculé C 59,28 % H 4,68 % N 8,13 % S 18,68 %

Trouvé C 59,15 % H 4,74 % N 8,06 % S 19,40 %

Spectre IR (KBr) 3100, 2960, 2820 $cm^{-1}$ bandes CH

1580, 1485, 1350, 1330, 1155, 1080, 905, 805, 710, et 655 $cm^{-1}$ bandes principales.

**EXEMPLE 9 : Chlorure de diméthyl-6-6, dihydro-5,6 4H 5H⊕ pyrrolo [1,2-a] thiéno [3,2-f] diazépinium [1,4].**

Une solution de 2 g (0,0093 moles) de chlorhydrate de (pyrrolyl-1)-2 amino méthyl-3 thiophène dans un mélange de 10 cm³ d'acétone, 50 cm³ de méthanol et 5 cm³ d'acide chlorhydrique concentré est chauffée au reflux pendant 3 heures. Après refroidissement le précipité formé par addition d'éther de pétrole est isolé par filtration, lavé avec de l'éther éthylique puis recristallisé dans l'isopropanol.

On obtient ainsi 1,5 g (59 %) de chlorure de diméthyl-6-6, dihydro-5,6 4H 5H⊕ pyrrolo [1,2-a] thiéno [3,2-f] diazépinium [1,4].

F = 250°c (isopropanol)

Microanalyse $C_{12}H_{15}N_2SCl$ M = 254,76

Calculé C 56,57 % H 5,93 % Cl 13,91 % N 10,94 %

Trouvé C 56,53 % H 6,07 % Cl 13,70 % N 10,94 %

Spectre IR (KBr) 2910, 2660 et 2620 $cm^{-1}$ bandes $NH_2^{\oplus}$

1575, 1560, 1480, 1440, 1340, 980, 875, et 745 $cm^{-1}$ bandes principales.

**EXEMPLE 10 : Hydroxyméthyl-6 dihydro-5,6 4H pyrrolo [1,2-a] thiéno [3,2-f] diazépine [1,4].**

On ajoute 0,60 g (0,015 moles) de borohydrure de sodium à une solution de 2 g (0,007 moles) d'éthoxycarbonyl-6 dihydro-5,6 4H pyrrolo [1,2-a] thiéno [3,2-f] diazépine [1,4] dans 30 cm³ de méthanol. Le milieu réactionnel est porté au reflux pendant 2 heures puis le méthanol est éliminé sous pression réduite. Le résidu est

repris par 100 cm³ d'eau et la solution aqueuse est extraite par de l'acétate d'éthyle.

Les phases organiques sont décolorées sur noir animal, séchées sur sulfate de magnésium et concentrées sous pression réduite.

Le précipité blanc obtenu est recristallisé dans de l'éther éthylique.

On obtient ainsi 1 g (60 %) d'hydroxyméthyl-6 dihydro-5,6 4H pyrrolo[1,2-a] thiéno [3,2-f] diazépine [1,4] sous forme de cristaux blancs.

F = 166°c (éther éthylique)

| | | |
|---|---|---|
| Microanalyse | $C_{11}H_{12}N_2OS$ | M = 220,28 |
| | Calculé | C 59,98 % H 5,49 % N 12,72 % S 14,55 % |
| | Trouvé | C 60,04 % H 5,58 % N 12,61 % S 14,38 % |

| | | |
|---|---|---|
| Spectre IR (KBr) | 3430 $cm^{-1}$ | bande OH |
| | 3260 cm-1 | bande NH |
| | 1580, 1480, 1400, 1340, 1320, 1160, 1040, 840 et 720 $cm^{-1}$ bandes principales. | |

L'éthoxycarbonyl-6 dihydro-5,6 4H pyrrolo[1,2-a] thiéno [3,2-f] diazépine [1,4] est obtenue par condensation en milieu éthanolique du chlorhydrate de (pyrrolyl-1)-2 aminométhyl-3 thiophène avec l'éthoxy-2 hydroxy-2 acétate d'éthyle.

**EXEMPLE 11 : Chlorure de cyclopropyl-6 dihydro-5,6 4H 5H$^{\oplus}$ pyrrolo [1,2-a] thiéno [3,2-f] diazépinium [1,4].**

On ajoute goutte à goutte une solution de 2 g (0,008 moles) de cyclopropyl-6 4H pyrrolo [1,2-a] thiéno [3,2-f] diazépine [1,4] dans 50 cm3 d'éther éthylique anhydre à une suspension de 0,30 g (0,008 moles) d'hydrure de lithium et d'aluminium dans 50 cm³ d'éther éthylique anhydre. Le milieu réactionnel est porté au reflux pendant 4 heures puis refroidi et versé lentement sur 100 cm³ d'eau.

La phase éthérée est décantée, lavée à l'eau , séchée sur sulfate de magnésium et concentrée sous pression réduite. On obtient ainsi la cyclopropyl-6 dihydro-5,6 4H pyrrolo [1,2-a] thiéno [3,2-f] diazépine [1,4] sous forme d'huile.

Cette huile est reprise dans 50 cm³ d'éther éthylique et l'on fait barboter pendant 1 minute un courant d'acide chlorhydrique gazeux. Le précipité est isolé par filtration, lavé avec de l'éther éthylique puis recristallisé dans l'isopropanol.

On obtient ainsi le chlorure de cyclopropyl-6 dihydro-5,6 4H 5H$^{\oplus}$ pyrrolo [1,2-a] thiéno [3,2-f] diazépinium [1,4] avec un rendement de 90 %.

F = 250°c (isopropanol)

| | | |
|---|---|---|
| Microanalyse | $C_{13}H_{15}N_2SCl$ | M = 266,78 |
| | Calculé | C 58,92 % H 5,67 % |
| | Trouvé | C 57,94 % H 5,62 % |

| | | |
|---|---|---|
| Spectre IR (KBr) | 2920, 2780, et 2560 $cm^{-1}$ | bandes $NH_2^{\oplus}$ |
| | 3120 cm-1 | bande CH |
| | 1570, 1480, 1440, 1340, 1140, 1100, 900 et 880 $cm^{-1}$ bandes principales. | |

La cyclopropyl-6 4H pyrrolo [1,2-a] thiéno [3,2-f] diazépine [1,4] est obtenue par condensation dans la pyridine du chlorure de l'acide cyclopropane carboxylique sur le (pyrrolyl-1)-2 aminométhyl-3 thiophène suivie d'une cyclisation de l'amide formée au moyen d'oxychlorure de phosphore.

**EXEMPLE 12 : Chlorure de méthyl-6 dihydro-5,6 4H 5H$^{\oplus}$ pyrrolo [1,2-a] thiéno [3,2-f] diazépinium [1,4] .**

Même protocole opératoire que pour l'exemple 11 en partant de méthyl-6 4H pyrrolo [1,2-a] thiéno [3,2-f] diazépine [1,4] . La méthyl-6 dihydro-5,6 4H pyrrolo [1,2-a] thiéno [3,2-f] diazépine [1,4] obtenue est purifiée par distillation sous vide. Le chlorhydrate correspondant est préparé de la même façon que dans l'exemple 11.

On obtient ainsi le chlorure de méthyl-6 dihydro-5,6 4H 5H$^{\oplus}$ pyrrolo [1,2-a] thiéno [3,2-f] diazépinium [1,4] avec un rendement de 90 %.

F = 242°c (isopropanol)

Microanalyse

| | | |
|---|---|---|
| Microanalyse | $C_{11}H_{13}N_2SCl$ | M = 240,75 |
| | Calculé | C 54,88 % H 5,44 % Cl 14,73 % S 13,32 % |
| | Trouvé | C 55,54 % H 5,64 % Cl 14,08 % S 12,83 % |

| | | |
|---|---|---|
| Spectre IR (KBr) | 2920, 2600 $cm^{-1}$ | bandes $NH_2^{\oplus}$ |
| | 1570, 1550, 1480, 1440, 1390, 1330, 1260, 1180, 1100, 1030 et 970 $cm^{-1}$ bandes principales. | |

La méthyl-6 4H pyrrolo [1,2-a] thiéno [3,2-f] diazépine [1,4] est obtenue par acylation du (pyrrolyl-1)-2 aminométhyl-3 thiophène dans un mélange d'acide et d'anhydride acétique suivie dune cyclisation de l'amide formée au moyen d'oxychlorure de phosphore.

**EXEMPLE 13 : Chlorure d'éthyl-6 dihydro-5,6 4H 5H$^{\oplus}$ pyrrolo [1,2-a] thiéno [3,2-f] diazépinium [1,4] .**

Même protocole opératoire que pour l'exemple 11 en partant d'éthyl-6 4H pyrrolo [1,2-a] thiéno [3,2-f] diazépine [1,4] . On obtient ainsi le chlorure d'éthyl-6 dihydro-5,6 4H 5H$^{\oplus}$ pyrrolo [1,2-a] thiéno [3,2-f] diazépinium [1,4] avec un rendement de 95 %.

F = 194°c (isopropanol)

| | | |
|---|---|---|
| Microanalyse | $C_{12}H_{15}N_2SCl$ | M = 254,77 |
| | Calculé | C 56,57 % H 5,93 % S 12,58 % |
| | Trouvé | C 56,81 % H 5,85 % S 12,69 % |

Spectre IR (KBr) 2920, 2740, 2540 $cm^{-1}$ bandes $NH_2^{\oplus}$
1570, 1480, 1380, 1260, 1170,1100, 900 et 860 $cm^{-1}$ bandes principales.

L'éthyl-6 4H pyrrolo [1,2-a] thiéno [3,2-f] diazépine [1,4] est obtenue par acylation du (pyrrolyl-1)-2 aminométhyl-3 thiophène dans la pyridine avec du chlorure de propionyle suivie dune cyclisation de l'amide formée au moyen d'oxychlorure de phosphore

**EXEMPLE 14 : Chlorure de diméthyl-2,3 dihydro-5,6 4H, 5H$^{\oplus}$ pyrrolo [1,2-a] thiéno [3,2-f] diazépinium [1,4] .**

**Stade I** : Diméthyl-4,5 (pyrrolyl-1)-2 cyano-3 thiophène

Une solution de 2 g ( 0, 0145 moles ) d'amino-2 cyano-3 diméthyl-4,5 thiophène et 2 $cm^3$ ( 0, 0160 moles ) de diméthoxy-2,5 tétrahydrofurane dans 25 $cm^3$ d'acide acétique est chauffée au reflux pendant 30 minutes. L'acide acétique est éliminé sous vide et l'huile brune résiduelle distillée.

On obtient ainsi 2 g (68 %) de diméthyl-4,5 (pyrrolyl-1)-2 cyano-3 thiophène sous forme de cristaux blancs.

F = 45°c

Microanalyse

Microanalyse $C_{11}H_{10}N_2S$ M = 344,44
Calculé C 65,32 % H 4,98 % N 13,85 % S 15,85 %
Trouvé C 65,15 % H 5,08 % N 13,63 % S 15,63 %

Spectre IR (KBr) 3170 et 2920 $cm^{-1}$ bandes CH
2220 $cm^{-1}$ bande CN
1520, 1425, 1090, 915 et 720 $cm^{-1}$bandes principales

**Stade II** : Diméthyl-4,5 (formyl-2 pyrrolyl-1)-2 cyano-3 thiophène,

13 g (0,064 moles) de diméthyl-4,5 (pyrrolyl-1)-2 cyano-3 thiophène en solution dans 20 $cm^3$ de DMF sont additionnés à température ambiante et en une seule fois à un complexe préalablement préparé à 0° c de 7,4 $cm^3$ (0,096 moles) de DMF et 8,8 $cm^3$ (0,096 moles) d'oxychlorure de phosphore. Le milieu réactionnel est agité 20 minutes à température ambiante puis 1 heure à 100°c. Après refroidissement le milieu réactionnel est versé sur 300 g de glace, agité pendant 20 minutes puis alcalinisé avec une solution de soude 6N. Le précipité formé est isolé par filtration, lavé à l'eau, séché puis recristallisé dans de l'éther éthylique.

On obtient ainsi 13,7 g (93 %) de diméthyl-4,5 (formyl-2 pyrrolyl-1)-2 cyano-3 thiophène sous forme de cristaux blancs.

F = 154°c (éther éthylique)

Microanalyse $C_{12}H_{10}N_2OS$ M = 230,25
Calculé C 62,61 % H 4,34 % N 12,17 % S 13,9 %
Trouvé C 62,74 % H 4,49 % N 12,09 % S 14,04 %

| | | |
|---|---|---|
| Spectre IR (KBr) | 3030, 2840 et 2750 $cm^{-1}$ | bandes CH |
| | 2220 $cm^{-1}$ | bande CN |

**Stade III** : Acide diméthyl-4,5 (formyl-2 pyrrolyl-1)-2 thiophène carboxylique-3.

Une solution de 5 g (0,022 moles) de diméthyl-4,5 (formyl-2 pyrrolyl-1)-2 cyano-3 thiophène dans 100 $cm^3$ d'hydroxyde de sodium 6N et 120 $cm^3$ d'éthanol est chauffée au reflux pendant 6 heures. L'éthanol est éliminé sous pression réduite, la solution aqueuse diluée avec 200 $cm^3$ d'eau et acidifiée avec une solution d'acide chlorhydrique 2N. Le précipité formé est isolé par filtration , lavé à l'eau, séché et recristallisé.

On obtient ainsi 3,5 g (64 %) d'acide diméthyl-4,5 (formyl-2 pyrrolyl-1)-2 thiophène carboxylique-3.

**Stade IV** : Diméthyl-4,5 (formyl-2 pyrrolyl-1) -2 carboxamido-3 thiophène.

On ajoute à 0°c 1,2 $cm^3$ (0,008 moles) de triéthylamine à une suspension de 2 g (0,008 moles) d'acide diméthyl-4,5 (formyl-2 pyrrolyl-1)-2 thiophène carboxylique-3 dans 200 $cm^3$ d'éther éthylique. Après 20 minutes d'agitation on ajoute 0,8 $cm^3$ (0,008 moles) de chloroformiate d'éthyle à la suspension maintenue à 0°c. Le mélange réactionnel est agité 20 minutes puis le précipité de chlorure de triéthylammonium formé est éliminé par filtration. On fait barboter de l'ammoniac dans la solution maintenue à 0°c et on isole par filtration le précipité qui se forme instantanément. Ce précipité est lavé à l'éther, séché et recristallisé.

On obtient ainsi 1,19 g (60 %) de diméthyl-4,5 (formyl-2 pyrrolyl-1)-2 carboxamido-3 thiophène.

**Stade V** : Diméthyl-2,3 hydroxy-6 oxo-4 dihydro-5,6 4H pyrrolo [1,2 -a] thiéno [3,2-f] diazépine [1,4] .

2 g (0,008 moles) de diméthyl-4,5 (formyl-2 pyrrolyl-1)-2 carboxamido-3 thiophène sont mis en suspension dans 20 $cm^3$ d'eau puis agités à température ambiante. Le précipité obtenu est isolé par filtration , lavé à l'eau, séché, puis recristallisé.

On obtient ainsi 1,29 g (65 %) de diméthyl-2,3 hydroxy-6 dihydro-5,6 oxo-4 4H pyrrolo [1,2-a] thiéno [3,2-f] diazépine [1,4] .

**Stade VI** : Chlorure de diméthyl-2,3 dihydro-5,6 4H $5H^{\oplus}$ pyrrolo [1,2-a] thiéno [3,2-f] diazépinium [1,4] .

Même protocole opératoire que pour le stade II de l'exemple 8 en partant de 5 g (0,02 moles) de diméthyl-2,3 hydroxy-6 oxo-4 dihydro-5,6 4H pyrrolo [1,2-a] thiéno [3,2-f] diazépine [1,4] .

L'huile obtenue est reprise dans 300 $cm^3$ d'éther éthylique puis l'on fait barboter un courant d'acide chlorhydrique gazeux. Le précipité obtenu est isolé par filtration, lavé avec de l'éther éthylique puis recristallisé.

On obtient ainsi 2,60 g (51 %) de chlorure de diméthyl-2,3 dihydro-5,6 4H $5H^{\oplus}$ pyrrolo [1,2-a] thiéno [3,2-f] diazépinium [1,4] .

F supérieur à 260°c.

TABLEAUX I

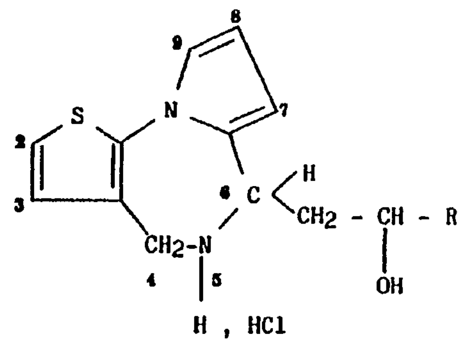

Tableau Ia (Les déplacements chimiques sont en ppm)

| Ex | R | $H_2$ | $H_3$ | $H_7$ | $H_8$ | $H_9$ | $NH_2^{\oplus}$ 5 | $CH_2$ 4 | $H_6$ | Autres protons |
|---|---|---|---|---|---|---|---|---|---|---|
| 1 | $CH_2CH_2CH_3$ | 7,45 | 7,12 | 6,44 | 6,36 | 7,26 | 9,97 | 4,16 | 4,21 | OH 4,38<br>$CH_2$ 3,54<br>CH 3,70<br>$2CH_2$ 1,36<br>$CH_3$ 0,86 |
| 2 | 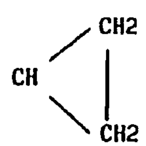 | 7,46 | 7,13 | 6,43 | 6,35 | 7,27 | 9,93 | 4,17 | 4,25 | OH 4,90<br>CH 3,68<br>CH 0,84<br>$CH_2$ 3,54<br>$2CH_2$ 0,38 et 0,20 |

(Suite du tableau Ia)

| Ex | R | $H_2$ | $H_3$ | $H_7$ | $H_8$ | $H_9$ | $NH_2^{\oplus}$ 5 | $CH_2$ 4 | $H_6$ | Autres protons |
|---|---|---|---|---|---|---|---|---|---|---|
| 3 | 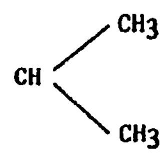 | 7,41 | 7,06 | 6,40 | 6,33 | 7,23 | 9,77 | 4,18 | 4,26 | OH 4,84<br>$CH_2$ 3,60<br>CH 3,72<br>CH 1,58<br>$2CH_2$ 0,84 |
| 4 | $C_6H_5$ | 7,48 | 7,14 | 6,61 | 6,41 | 7,30 | 9,86 | 4,28 | 4,54 | OH 5,66<br>$CH_2$ 3,60<br>CH 3,72<br>$C_6H_5$ 7,38; 7,36;et 7,32 |
| 5 | $CH_3$ | 7,45 | 7,12 | 6,49 | 6,35 | 7,27 | 10 | 3,96 | 4,25 | OH 4,84<br>CH 3,61<br>$CH_2$ 3,37<br>$CH_3$ 1,07 |

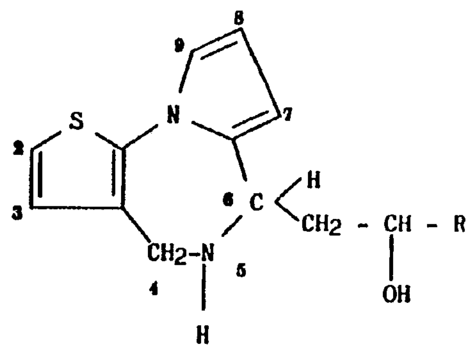

Tableau $I_b$ (Les déplacements chimiques sont en ppm)

| Ex | R | $H_2$ | $H_3$ | $H_7$ | $H_8$ | $H_9$ | NH 5 | $H_6$ | $CH_2$ 4 | Autres protons |
|---|---|---|---|---|---|---|---|---|---|---|
| 6 | $CH_3$ | 6,90 | 6,70 | 6,06 | 6,19 | 7,00 | 6,95 | 4,11 | 4,24 | CH 4,02<br>$CH_3$ 1,25<br>$CH_2$ 1,87 - 2,04 |
| 7 | $C_6H_5$ | 6,91 | 6,62 | 6,06 | 6,15 | 6,96 | 6,95 | 4,35 | 4,33 | CH 3,74<br>$CH_2$ 2,49<br>$C_6H_5$ 7,37 |

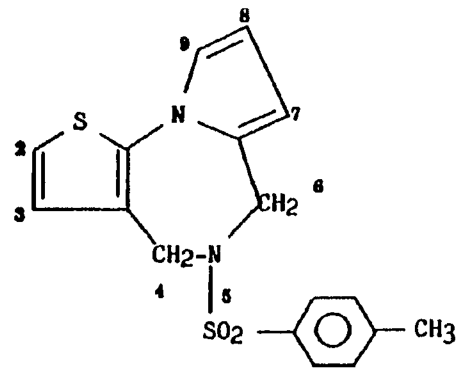

**Tableau Ic (Les déplacements chimiques sont en ppm)**

| Ex | $H_3$ | $H_7$ | $H_8$ | $H_9$ | $CH_2$ 4 | $CH_2$ 6 | Autres protons |
|---|---|---|---|---|---|---|---|
| 8 | 6,87 | 6,16 | 6,12 | 6,87 | 4,42 | 3,37 | $CH_3$ 2,33<br>$C_6H_4$ 7,52 et 7,26 |

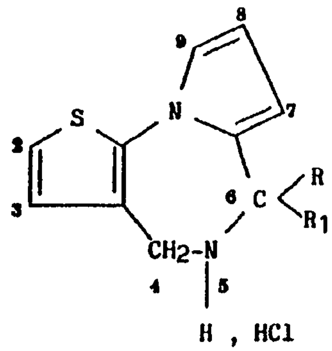

Tableau Id (Les déplacements chimiques sont en ppm)

| Ex | R | $R_1$ | $H_2$ | $H_3$ | $H_7$ | $H_8$ | $H_9$ | $CH_2$ 4 | Autres protons |
|---|---|---|---|---|---|---|---|---|---|
| 9 | CH3 | $CH_3$ | 7,30 | 6,93 | 6,20 | 6,30 | 7,10 | 4,1 | $CH_3$ 1,6<br>$NH_2^{\oplus}$ 10,3 |
| 11 | H | 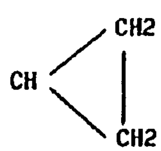 | 7,38 | 7,06 | 6,35 | 6,60 | 7,23 | 4,25<br>3,70 | H6 2,5<br>cyclopropyl 1,55;0,7;0,32<br>$NH_2^{\oplus}$ 10,09 |

(Suite du tableau Id)

| Ex | R | $R_1$ | $H_2$ | $H_3$ | $H_7$ | $H_8$ | $H_9$ | $CH_2$ 4 | Autres protons |
|---|---|---|---|---|---|---|---|---|---|
| 12 | H | $CH_3$ | 7,42 | 7,10 | 6,34 | 6,44 | 7,25 | 4,28<br>3,63 | $H_6$ 4,08<br>$CH_3$ 1,65<br>$NH_2^{\oplus}$ 10,28 |
| 13 | H | $C_2H_5$ | 7,41 | 7,09 | 6,34 | 6,44 | 7,24 | 4,22<br>3,61 | $H_6$ 3,90<br>$CH_2(C_2H_5)$ à 2,05<br>$CH_3$ $(C_2H_5)$ à 0,9 |

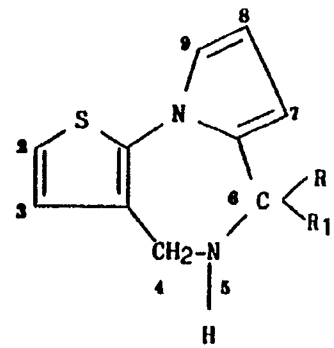

Tableau Ie ( Les déplacements chimiques sont en ppm)

| Ex | R | $R_1$ | $H_2$ | $H_3$ | $H_7$ | $H_8$ | $H_9$ | $CH_2$ | Autres protons |
|---|---|---|---|---|---|---|---|---|---|
| 10 | H | $CH_2OH$ | 7,12 | 6,78 | 6,04 | 6,11 | 7,01 | 4,00 | $CH_2$ 3,65<br>OH 4,71<br>$H_6$ 3,8 |

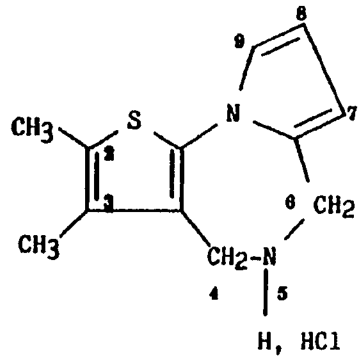

**Tableau If ( Les déplacements chimiques sont en ppm)**

| Ex | $H_7$ | $H_8$ | $H_9$ | Autres protons |
|---|---|---|---|---|
| 14 | 6,39 | 6,27 | 7,18 | $CH_2$ : 3,95 (s) $CH_2$ : 4,10 (s) $CH_3$ : 2,06 (s) $CH_2$ : 2,25 (s) $NH_2^{\oplus}$ : 10,05 |

**EXEMPLE 15 : Comprimé dosé à 3 mg de chlorure de diméthyl-2,3 dihydro-5,6 4H 5H⊕ pyrrolo [1,2-a] thiéno [3,2-f] diazépinium [1,4].**

- Chlorure de diméthyl-2,3 dihydro-5,6 4H 5H⊕ pyrrolo [1,2 -a] thiéno [3,2 -f] diazépinium [1,4] ---------- 3 g
- Amidon de blé ---------- 110 g
- Amidon de maïs ---------- 85 g
- Stéarate de magnésium ---------- 15 g
- Talc ---------- 20 g

pour 1000 comprimés à 3 mg de principe actif.

**EXEMPLE 16 : Etude pharmacologique. Mise en évidence des propriétés antihypoxiques**

**a) Principe**

Lors du vieillissement, la baisse des réserves intrinsèques cérébrales se traduit par l'incapacité à répondre à toute nouvelle demande métabolique.

Lors d'un accident vasculaire cérébral, la vulnérabilité sélective neuronale apparait dans les cellules qui ont la plus forte demande métabolique.

Le point commun entre ces pathologies est le déficit d'utilisation d'oxygène qui mène le plus souvent à la déviation du métabolisme vers l'anaérobie.

De telles agressions peuvent être reproduites expérimentalement et de façon aigüe par hypoxie pour la recherche de nouveaux composés cérébro protecteurs.

En effet, l'analogie entre l'hypoxie et les désordres cérébraux liés au vieillissement ou à l'insuffisance circulatoire s'exprime notamment par :

– la baisse des réserves énergétiques,
– la moindre résistance au stress,
– la baisse de la synthèse oxygéno dépendante des neuro transmetteurs,
– la chute des capacités mnésiques.

Les composés de la présente invention ont donc été testés sur leur capacité à prolonger la survie du tissu cérébral chez la souris.

**b) Méthodologie**

Des souris CD1 (Charles River) de sexe mâle sont soumises à une hypoxie aigüe de type hypobare 30 minutes après avoir reçu par voie intra péritonéale le composé étudié. Pour cela elles sont placées dans une enceinte où la pression barométrique peut être abaissée rapidement à une valeur de 160 mbar.

Le renouvellement d'air assuré durant l'épreuve permet de provoquer une hypoxie et non une asphyxie ou une anoxie qui pourrait faire appel à une protection d'origine vasculaire.

La mort cérébrale est obtenue en 15 secondes environ après l'obtention de la pression hypoxique.

Le temps moyen de survie d'un lot traité est comparé à celui d'un lot témoin ne recevant que le solvant d'injection.

**c) Résultats**

Les composés de l'invention n'entrainent pas d'effet neuro comportementaux de type sédation contrairement au diazepam. Ils s'opposent significativement à l'hypoxie cérébrale.

Hypoxie aigue : Pourcentage d'augmentation du temps de survie cérébrale.

| Exemple | mg/Kg Ip | | | |
|---|---|---|---|---|
| | 3 | 10 | 30 | 100 |
| 2 | +12 | +17 | +22 | +94 |
| 3 | +9 | +11 | +19 | +82 |
| 5 | +11 | +15 | +27 | +125 |
| 14 | +35 | +54 | +128 | +250 |

## Revendications

**1)** Pyrrolo [1,2 -a] thiéno [3,2 -f] diazépine [1,4] de formule générale **(I)** :

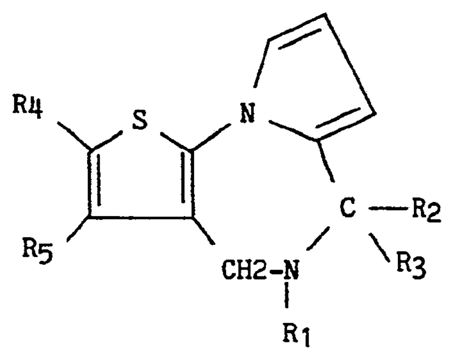

(I)

dans laquelle

– $R_1$ représente un atome d'hydrogène, un groupement alkyle inférieur de 1 à 6 atomes de carbone ramifié ou non éventuellement substitué par des groupements hydroxy, alkoxy, cycloalkyle, aryle, un groupement alkylsulfonyle ou arylsulfonyle éventuellement substitué sur le cycle aromatique par un ou plusieurs groupements alkyles inférieurs ou des halogènes

– $R_4$ et $R_5$ représentent chacun indépendamment l'un de l'autre un atome d'hydrogène, un groupement alkyle inférieur de 1 à 6 atomes de carbone ramifié ou non éventuellement substitué par des groupements hydroxy, alkoxy, cycloalkyle, aryle substitué ou non, un groupement aryle ou arylalkyle éventuellement substitué (le terme substitué associé aux expressions aryle ou aralkyle signifie que les noyaux aromatiques peuvent être substitués par un ou des groupements alkyle, nitro, alkoxy, trifluorométhyle ou halogène)

– $R_2$ et $R_3$ représentent chacun indépendamment l'un de l'autre un atome d'hydrogène, un groupement aryle ou aralkyle éventuellement substitué, un groupement alkyle inférieur de 1 à 6 atomes de carbone ramifié ou non éventuellement substitué par un ou des groupements hydroxy, alkoxy, oxo, cycloalkyle, aryle substitué ou non, ou un groupement -NR'R" pour lequel R' et R" représentent chacun indépendamment l'un de l'autre un atome d'hydrogène, un groupement -COR"' (R"' représente soit un phényl éventuellement substitué, soit un indolyl, soit un groupement anilino éventuellement substitué), un groupement alkyle inférieur, ou forment ensemble avec l'atome d'azote qui les portent un système hétérocyclique mono ou bicyclique comportant ou non un autre hétéroatome et éventuellement substitué par un groupement alkyle inférieur, aryle ou aralkyle éventuellement substitués (le terme substitué associé aux expressions aryle, phényle, anilino et aralkyle signifie que les noyaux aromatiques peuvent être substitués par un ou plusieurs groupements alkyle, nitro, alkoxy, trifluorométhyle ou halogène)

avec la réserve que, lorsque $R_1 = R_4 = R_5$ : H et l'un des deux substituants $R_2$ ou $R_3$ est un atome d'hydrogène, l'autre substituant ne peut représenter ni un atome d'hydrogène, ni un groupement méthoxy, ni un groupement aryle éventuellement substitué

– leurs isomères, diastéroisomères, énantiomères
– leurs sels d'addition à un acide minéral ou organique, pharmaceutiquement acceptable.

**2)** Composés selon la revendication 1) pour lesquels $R_4$ et $R_5$ représentent soit un atome d'hydrogène , soit un groupement alkyle inférieur de 1 à 6 atomes de carbone, ainsi que leurs isomères, diastéréoisomères, énantiomères et leurs sels d'addition à un acide minéral ou organique pharmaceutiquement acceptable.

**3)** Composé selon les revendications 1) et 2) qui est l'(hydroxy-2 pentyl)-6 dihydro-5,6 4H pyrrolo [1,2-a] thiéno [3,2-f] diazépine [1,4] ainsi que ses isomères isolés ou sous forme de mélange et leurs sels d'addition à un acide pharmaceutiquement acceptable.

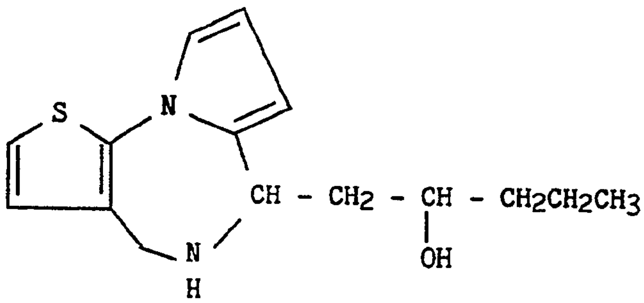

**4)** Composé selon les revendications 1) et 2) qui est la (cyclopropyl-2 hydroxy-2 éthyl)-6 dihydro-5,6 4H pyrrolo [1,2-a] thiéno[3,2-f] diazépine [1,4] ainsi que ses isomères isolés ou sous forme de mélange et leurs sels d'addition à un acide pharmaceutiquement acceptable.

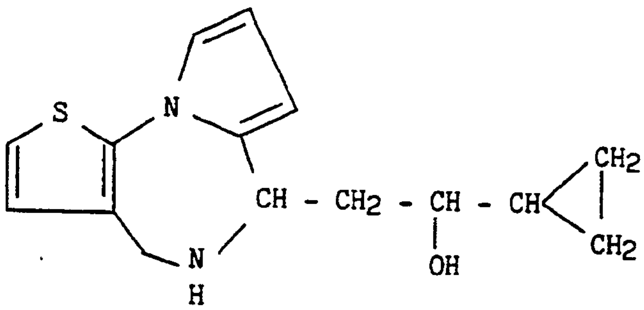

**5)** Composé selon les revendications 1) et 2) qui est l'(hydroxy-2 méthyl-3 butyl)-6 dihydro-5,6 4H pyrrolo [1,2-a] thiéno [3,2-f] diazépine [1,4] ainsi que ses isomères isolés ou sous forme de mélange et leurs sels d'addition à un acide pharmaceutiquement acceptable.

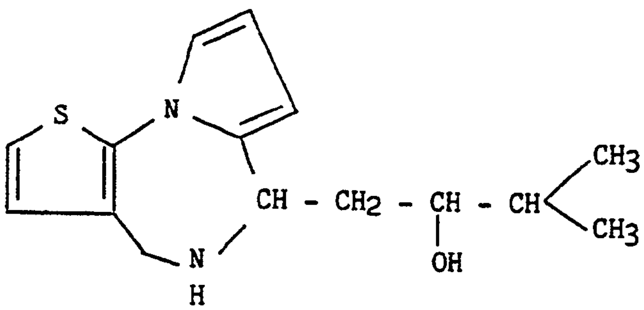

**6)** Composé selon les revendications 1) et 2) qui est la (phényl-2 hydroxy-2 éthyl)-6 dihydro-5,6 4H pyrrolo [1,2-a] thiéno [3,2-f] diazépine [1,4] ainsi que ses isomères isolés ou sous forme de mélange et leurs sels d'addition à un acide pharmaceutiquement acceptable.

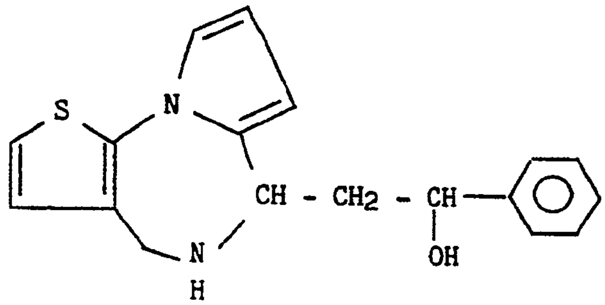

**7)** Composé selon les revendications 1) et 2) qui est l'(hydroxy-2 propyl)-6 dihydro-5,6 4H pyrrolo [1,2-a] thiéno [3,2-f] diazépine [1,4] ainsi que ses isomères isolés ou sous forme de mélange et leurs sels d'addition à un acide pharmaceutiquement acceptable.

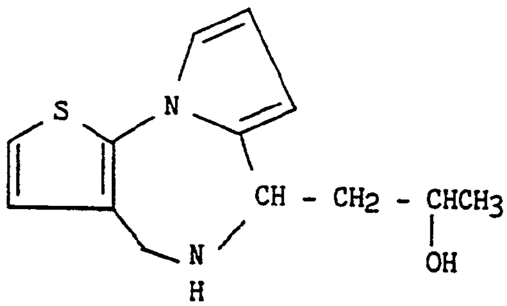

**8)** Composé selon les revendications 1) et 2) qui est le para tolyl sulfonyl-5 dihydro-5,6 4H pyrrolo [1,2-a] thiéno [3,2-f] diazépine [1,4].

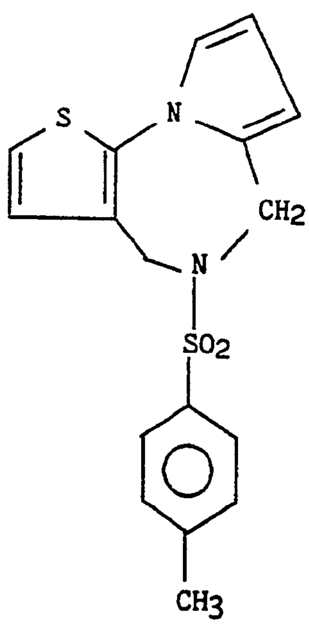

**9)** Composé selons les revendications 1) et 2) qui est la diméthyl-6,6 dihydro-5,6 4H pyrrolo [1,2-a] thiéno [3,2-f] diazépine [1,4] ainsi que ses sels d'addition à un acide pharmaceutiquement acceptable.

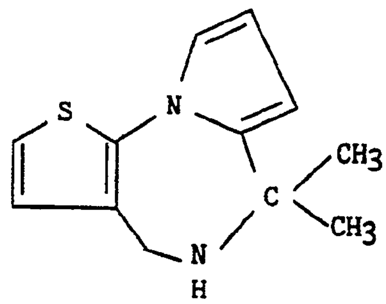

**10)** Composé selon les revendications 1) et 2) qui est l'hydroxyméthyl-6 dihydro-5,6 4H pyrrolo [1,2-a] thiéno [3,2-f] diazépine [1,4] ainsi que ses isomères isolés ou sous forme de mélange et leurs sels d'addition à un acide pharmaceutiquement acceptable.

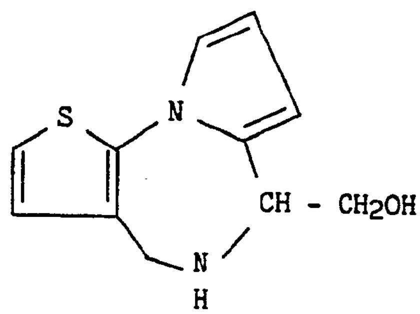

**11)** Composé selon les revendications 1) et 2) qui est la cyclopropyl-6 dihydro-5,6 4H pyrrolo [1,2-a] thiéno [3,2-f] diazépine [1,4] ainsi que ses isomères isolés ou sous forme de mélange et leurs sels d'addition à un acide pharmaceutiquement acceptable.

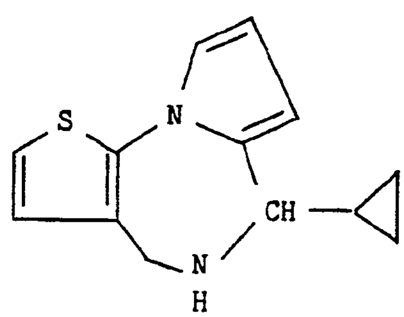

**12)** Composé selon les revendications 1) et 2) qui est la méthyl-6 dihydro-5,6 4H pyrrolo [1,2-a] thiéno [3,2-f] diazépine [1,4] ainsi que ses isomères isolés ou sous forme de mélange et leurs sels d'addition à un acide pharmaceutiquement acceptable.

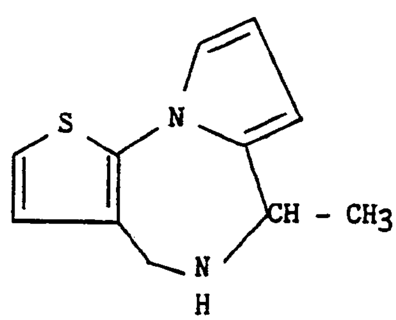

**13)** Composé selon les revendications 1) et 2) qui est l'éthyl-6 dihydro-5,6 4H pyrrolo [1,2-a] thiéno [3,2-f] diazépine [1,4] ainsi que ses isomères isolés ou sous forme de mélange et leurs sels d'addition à un acide

pharmaceutiquement acceptable.

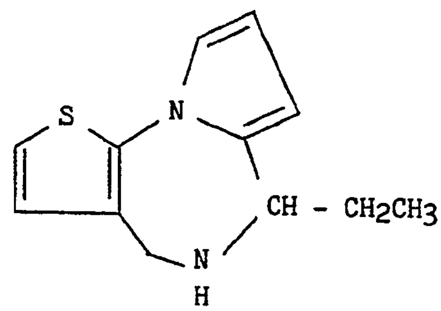

**14)** Composé selon les revendications 1) et 2) qui est la diméthyl-2,3 dihydro-5,6 4H pyrrolo [1,2-a] thiéno [3,2-f] diazépine [1,4] ainsi que ses sels d'addition à un acide pharmaceutiquement acceptable.

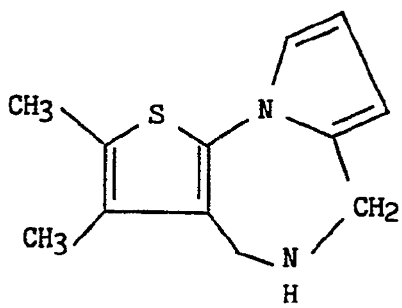

**15)** Procédé de préparation des dérivés de la revendication 1) qui peut varier selon la nature des substituants $R_1$, $R_2$, $R_3$, $R_4$ et $R_5$ :

- soit l'on part d'un dérivé de formule générale **(II)** :

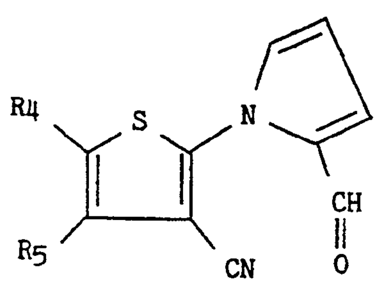

(II)

dans laquelle $R_4$ et $R_5$ ont la même signification que dans le dérivé de formule générale **(I)** que l'on fait réagir dans un mélange d'une solution aqueuse d'hydroxyde de sodium, d'alcool et de peroxyde d'hydrogène avec une méthyle cétone de formule générale **(III)** :

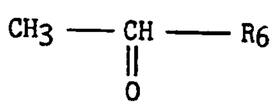

(III)

dans laquelle $R_6$ est un groupement cycloalkyle, alkyle inférieur de 1 à 6 atomes de carbone ramifié ou non éventuellement substitué par des groupements alkoxy, cycloalkyle, aryle, de manière à obtenir la dihydro-5,6 oxo-4 4H pyrrolo [1,2-a] thiéno [3,2-f] diazépine [1,4] de formule générale **(IV)** :

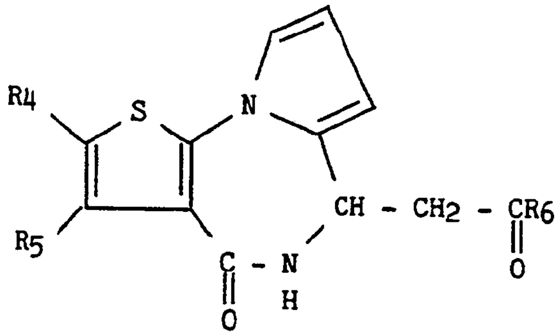

(IV)

dans laquelle $R_6$ a la même signification que dans la formule générale **(III)** et $R_4$ et $R_5$ la même signification que dans le dérivé de formule générale **(I)** que l'on réduit en milieu aprotique par un hydrure métallique mixte de manière à obtenir la dihydro-5,6 4H pyrrolo [1,2-a] thiéno [3,2-f] diazépine [1,4] de formule générale générale **(V)** :

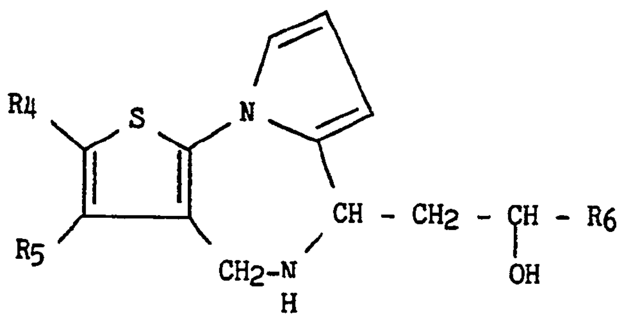

(V)

dans laquelle $R_4$, $R_5$ et $R_6$ ont la même signification que dans la formule **(III)** que l'on peut traiter en solution éthérée par de l'acide chlorhydrique gazeux pour obtenir le chlorure de dihydro-5,6 4H 5H⊕ pyrrolo [1,2-a] thiéno [3,2 f] diazépinium [1,4] de formule générale **(VI)**:

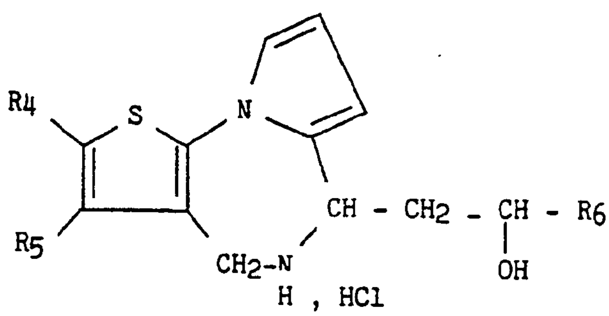

(VI)

dans laquelle $R_4$, $R_5$ et $R_6$ ont la même signification que la formule **(III)**,
- soit l'on part du dérivé de formule générale **(VII)** :

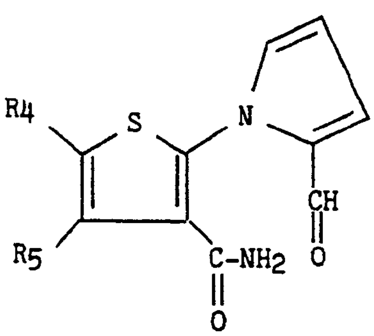

(VII)

dans laquelle $R_4$ et $R_5$ ont la même signification que dans le dérivé de formule générale **(I)** que l'on fait réagir en suspension dans l'eau avec une amine tertiaire de manière à obtenir le dérivé de formule générale **(VIII)** :

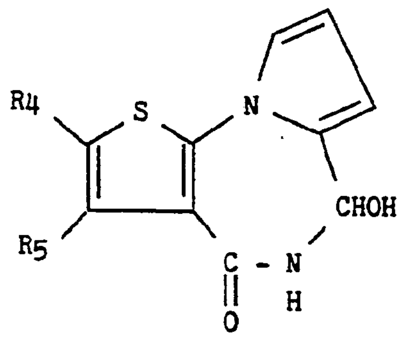

(VIII)

dans laquelle $R_4$ et $R_5$ ont la même signification que dans le dérivé de formule générale **(I)** que l'on réduit en milieu aprotique par un hydrure métallique mixte de manière à obtenir le dérivé de formule générale **(IX)** :

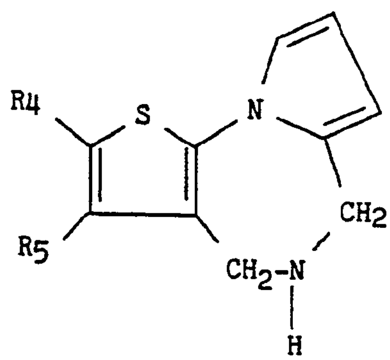

(IX)

dans laquelle $R_4$ et $R_5$ ont la même signification que dans le dérivé de formule générale **(I)**, que l'on fait réagir en présence d'amine avec un dérivé halogéné de formule générale **(X)** :

$R_1Cl$ **(X)**

dans laquelle $R_1$ a la même signification que dans le dérivé de formule générale **(I)** de manière à obtenir le dérivé de formule générale **(XI)** :

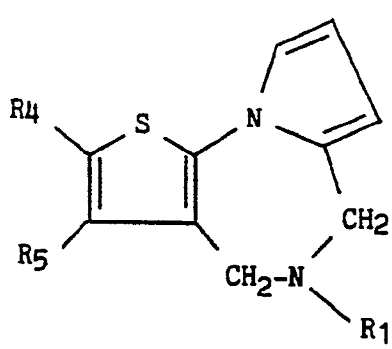

(XI)

dans laquelle $R_1$ ,$R_4$ et $R_5$ ont la même signification que dans le dérivé de formule générale **(I)**,

- soit l'on part du dérivé de formule générale **(XII)** :

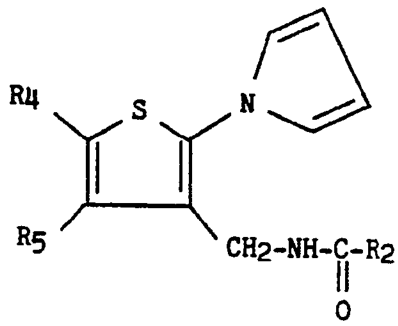

**(XII)**

dans laquelle $R_2$, $R_4$ et $R_5$ ont la même signification que dans le dérivé de formule générale **(I)** que l'on cyclise avec de l'oxychlorure de phosphore de manière à obtenir le dérivé de formule générale **(XIII)** :

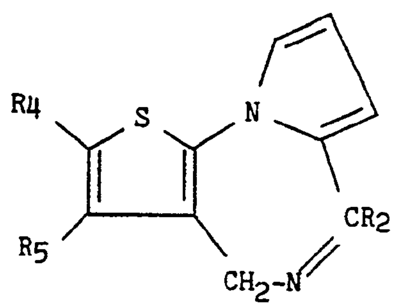

**(XIII)**

dans laquelle $R_2$, $R_4$ et $R_5$ ont la même signification que dans le dérivé de formule générale **(I)** que l'on réduit en milieu aprotique par un hydrure métallique mixte de manière à obtenir la dihydro-5,6 4H pyrrolo [1,2-a] thiéno [3,2-f] diazépine [1,4] de formule générale **(XIV)** :

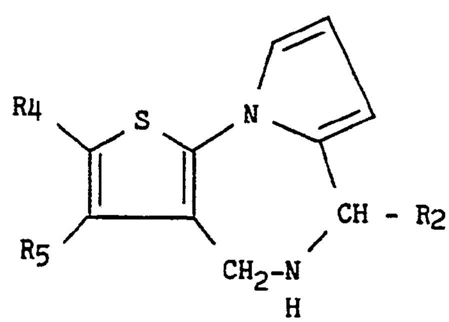

**(XIV)**

dans laquelle $R_2$, $R_4$ et $R_5$ ont la même signification que dans le dérivé de formule générale **(I)** que l'on peut traiter en solution éthérée par de l'acide chlorhydrique gazeux pour obtenir le chlorure de dihydro-5,6 4H $5H^{\oplus}$ pyrrolo [1,2-a] thiéno [3,2-f] diazépinium [1,4] de formule générale **(XV)** :

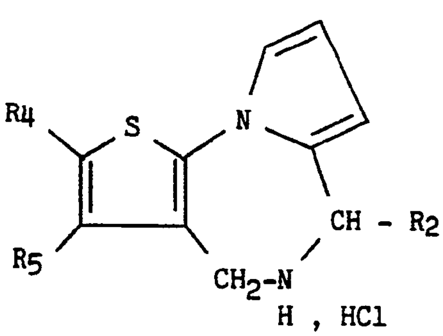

**(XV)**

dans laquelle $R_2$, $R_4$ et $R_5$ ont la même signification que dans le dérivé de formule générale **(I)**,

- soit l'on part du dérivé de formule générale **(XVI)** :

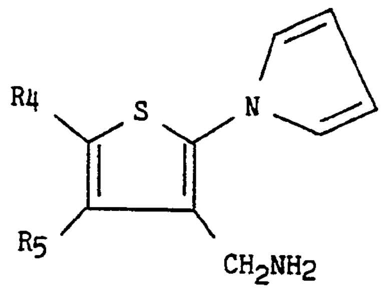

(XVI)

dans laquelle $R_4$ et $R_5$ ont la même signification que dans le dérivé de formule générale **(I)** que l'on fait réagir dans de l'alcool au reflux avec un aldéhyde de formule générale **(XVII)** :

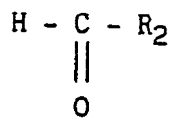

(XVII)

dans laquelle $R_2$ a la même signification que dans le dérivé de formule générale **(I)** de manière à obtenir le composé de formule générale **(XVIII)** :

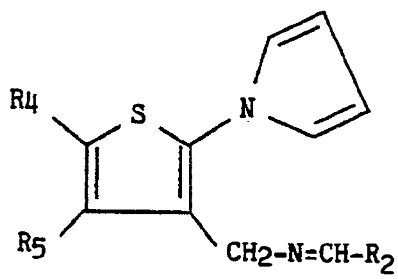

(XVIII)

dans laquelle $R_2$ $R_4$ et $R_5$ ont la même signification que dans le dérivé de formule générale **(I)** que l'on traite en solution éthérée par de l'acide chlorhydrique gazeux de manière à obtenir le chlorure de dihydro-5,6 4H 5H⊕ pyrrolo [1,2-a] thiéno [3,2-f] diazépinium [1,4] de formule générale **(XV)** dans laquelle $R_2$, $R_4$ et $R_5$ ont la même signification que dans le dérivé de formule générale **(I)**,

- soit l'on part à partir du dérivé de formule générale **(XIX)** :

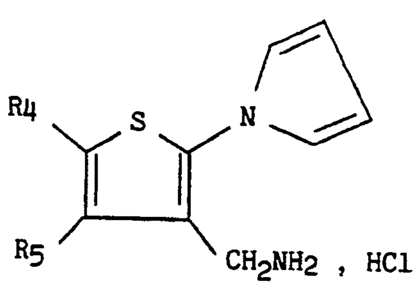

(XIX)

dans laquelle $R_4$ et $R_5$ ont la même signification que dans le dérivé de formule générale **(I)** que l'on fait réagir au reflux dans de l'alcool et en présence d'acide chlorhydrique concentré avec une cétone de formule générale **(XX)** :

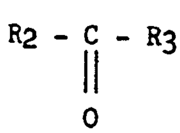

(XX)

dans laquelle $R_2$ et $R_3$ ont la même signification que dans le dérivé de formule générale **(I)** de manière à obtenir le chlorure de dihydro-5,6 4H 5H⊕ pyrrolo [1,2-a] thiéno [3,2-f] diazépinium [1,4] de formule générale **(XXI)** :

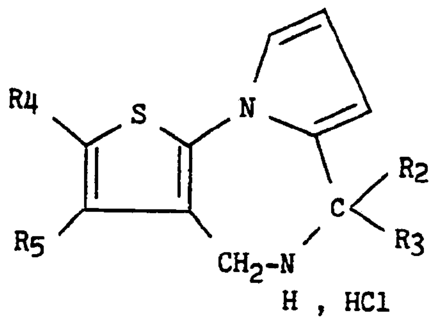

(XXI)

dans laquelle $R_2$, $R_3$, $R_4$ et $R_5$ ont la même signification que dans le dérivé de formule générale **(I)**,

- soit l'on part du dérivé de formule générale **(XIX)** dans laquelle $R_4$ et $R_5$ ont la même signification que dans le dérivé de formule générale **(I)** que l'on fait réagir en milieu alcoolique avec de l'éthoxy-2 hydroxy-2 acétate d'éthyle de manière à obtenir le dérivé de formule générale **(XXII)** :

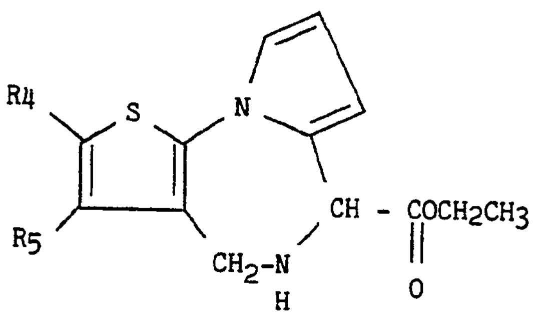

(XXII)

dans laquelle $R_4$ et $R_5$ ont la même signification que dans le dérivé de formule générale **(I)** que l'on réduit en solution alcoolique par un hydrure métallique mixte de manière à obtenir l'hydroxyméthyl-6 dihydro-5,6 4H pyrrolo [1,2-a] thiéno [3,2-f] diazépine [1,4] de formule générale **(XXIII)** :

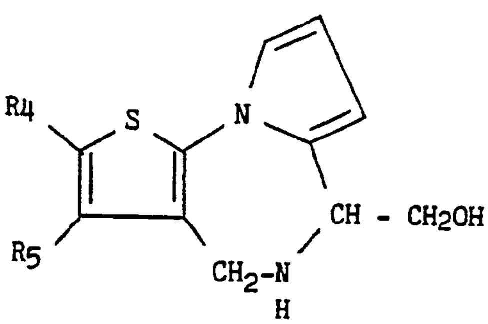

(XXIII)

dans laquelle $R_4$ et $R_5$ ont la même signification que dans le dérivé de formule générale **(I)**,

- soit l'on part du dérivé de formule générale **(VIII)** dans laquelle $R_4$ et $R_5$ ont la même signification que dans le dérivé de formule générale **(I)** que l'on fait réagir au reflux de l'acétonitrile avec une amine de formule générale **(XXIV)** :

HNR'R" **(XXIV)**

dans laquelle R' et R" ont la même signification que dans le dérivé de formule générale **(I)** de manière à obtenir le dérivé de formule générale **(XXV)** :

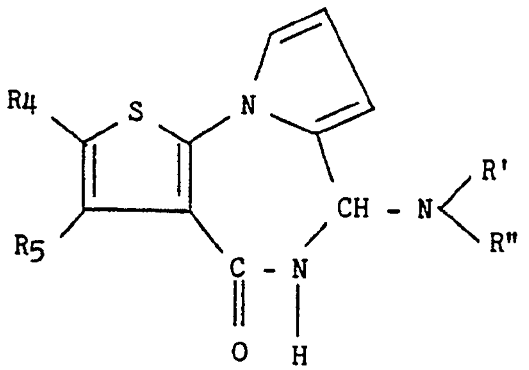

(XXV)

dans laquelle $R_4$, $R_5$, R′ et R″ ont la même signification que dans le dérivé de formule générale **(I)** que l'on réduit en milieu aprotique par un hydrure métallique mixte de manière à obtenir le dérivé de formule générale **(XXVI)** :

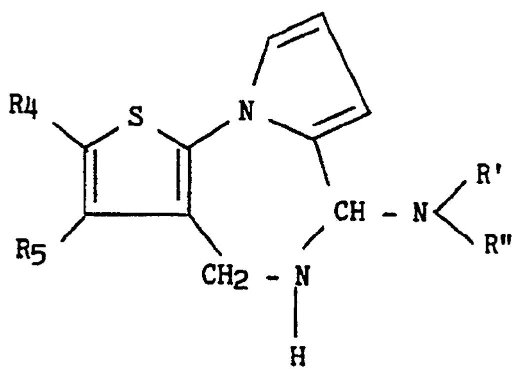

(XXVI)

dans laquelle $R_4$, $R_5$, R′ et R″ ont la même signification que dans le dérivé de formule générale (I), étant entendu que les composés de formule générale **V,VI,IX,XI,XIV,XV,XXI,XXII,XXIII** et **XXVI** sont des cas particuliers des dérivés de formule générale **(I)**.

**16)** Compositions pharmaceutiques contenant comme principe actif un dérivé selon les revendications 1) à 14) seul ou en combinaison avec un ou plusieurs excipients ou véhicules inertes non toxiques pharmaceutiquement acceptables.

**17)** Compositions pharmaceutiques selon la revendication 16) présentées sous une forme convenant notamment pour le traitement des accidents vasculaires cérébraux, du vieillissement cérébral normal ou pathologique des syndromes ischémiques, de l'hypercholestérolémie de l'hyperlipidémie, de l'hypertriglycéridémie, de l'hyperglycémie, de l'hypertension et des maladies qui leurs sont liées.

**Revendications pour l'Etat contractant suivant: ES**

**1)** Procédé de préparation de pyrrolo [1,2 -a] thiéno [3,2 -f] diazépine [1,4] de formule générale **(I)** :

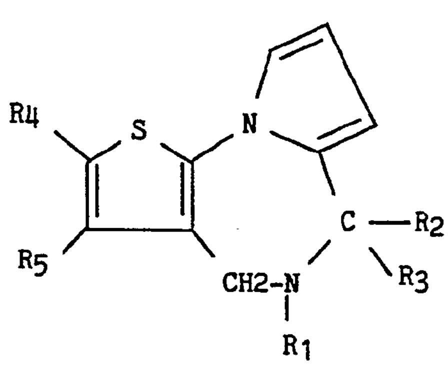

(I)

dans laquelle

– $R_1$ représente un atome d'hydrogène, un groupement alkyle inférieur de 1 à 6 atomes de carbone ramifié ou non éventuellement substitué par des groupements hydroxy, alkoxy, cycloalkyle, aryle, un groupement alkylsulfonyle ou arylsulfonyle éventuellement substitué sur le cycle aromatique par un ou plusieurs groupements alkyles inférieurs ou des halogènes

– $R_4$ et $R_5$ représentent chacun indépendamment l'un de l'autre un atome d'hydrogène, un groupement alkyle inférieur de 1 à 6 atomes de carbone ramifié ou non éventuellement substitué par des groupements hydroxy, alkoxy, cycloalkyle, aryle substitué ou non, un groupement aryle ou arylalkyle éventuellement substitué (le terme substitué associé aux expressions aryle ou aralkyle signifie que les noyaux aromatiques peuvent être substitués par un ou des groupements alkyle, nitro, alkoxy, trifluorométhyle ou halogène)

– $R_2$ et $R_3$ représentent chacun indépendamment l'un de l'autre un atome d'hydrogène, un groupement aryle ou aralkyle éventuellement substitué, un groupement alkyle inférieur de 1 à 6 atomes de carbone ramifié ou non éventuellement substitué par un ou des groupements hydroxy, alkoxy, oxo, cycloalkyle, aryle substitué ou non, ou un groupement -NR'R" pour lequel R' et R" représentent chacun indépendamment l'un de l'autre un atome d'hydrogène, un groupement -COR"' (R"' représente soit un phényl éventuellement substitué, soit un indolyl, soit un groupement anilino éventuellement substitué), un groupement alkyle inférieur, ou forment ensemble avec l'atome d'azote qui les portent un système hétérocyclique mono ou bicyclique comportant ou non un autre hétéroatome et éventuellement substitué par un groupement alkyle inférieur, aryle ou aralkyle éventuellement substitués (le terme substitué associé aux expressions aryle, phényle, anilino et aralkyle signifie que les noyaux aromatiques peuvent être substitués par un ou plusieurs groupements alkyle, nitro, alkoxy, trifluorométhyle ou halogène)

ainsi que de leurs isomères, diastéroisomères, énantiomères et de leur sel d'addition à un acide minéral ou organique, pharmaceutiquement acceptable,

avec la réserve que, lorsque $R_1 = R_4 = R_5 = H$ et l'un des deux substituants $R_2$ ou $R_3$ est un atome d'hydrogène, l'autre substituant ne peut représenter ni un atome d'hydrogène, ni un groupement méthoxy, ni un groupement aryle éventuellement substitué caractérisé en ce que :

- soit l'on part d'un dérivé de formule générale **(II)** :

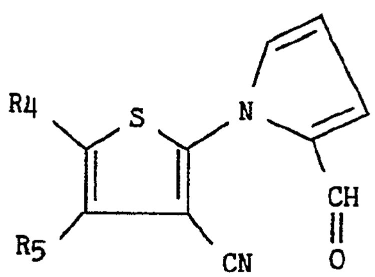

**(II)**

dans laquelle $R_4$ et $R_5$ ont la même signification que dans le dérivé de formule générale **(I)** que l'on fait réagir dans un mélange d'une solution aqueuse d'hydroxyde de sodium, d'éthanol et de peroxyde d'hydrogène avec une méthyle cétone de formule générale **(III)** :

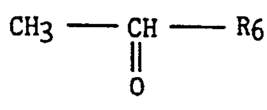

**(III)**

dans laquelle $R_6$ est un groupement cycloalkyle, alkyle inférieur de 1 à 6 atomes de carbone ramifié ou non éventuellement substitué par des groupements alkoxy, cycloalkyle, aryle, de manière à obtenir la dihydroxy-5,6 oxo-4 4H pyrrolo [1,2-a] thiéno [3,2-f] diazépine [1,4] de formule générale **(IV)** :

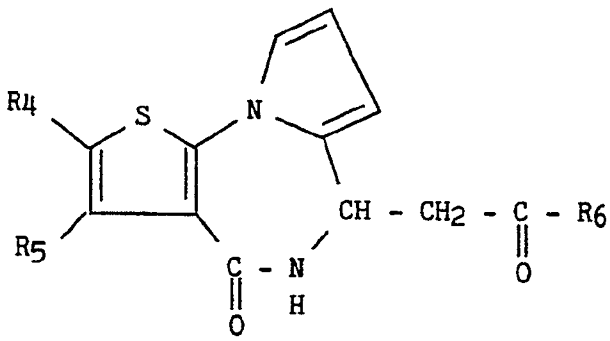

(IV)

dans laquelle $R_6$ a la même signification que dans la formule générale **(III)** et $R_4$ et $R_5$ la même signification que dans le dérivé de formule générale **(I)** que l'on réduit en milieu aprotique par un hydrure métallique mixte de manière à obtenir la dihydro-5,6 4H pyrrolo [1,2-a] thiéno [3,2-f] diazépine [1,4] de formule générale **(V)** :

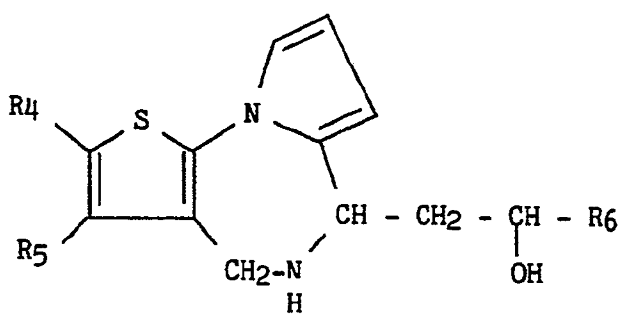

(V)

dans laquelle $R_4$, $R_5$ et $R_6$ ont la même signification que dans la formule **(III)** que l'on peut traiter en solution éthérée par de l'acide chlorhydrique gazeux pour obtenir le chlorure de dihydro-5,6 -4H -5H$^{\oplus}$ pyrrolo [1,2-a] thiéno [3,2-f] diazépinium [1,4] de formule générale **(VI)** :

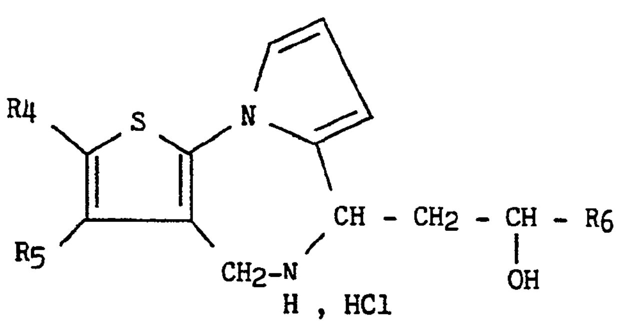

(VI)

dans laquelle $R_4$, $R_5$ et $R_6$ ont la même signification que la formule **(III)**,

- soit l'on part du dérivé de formule générale **(VII)** :

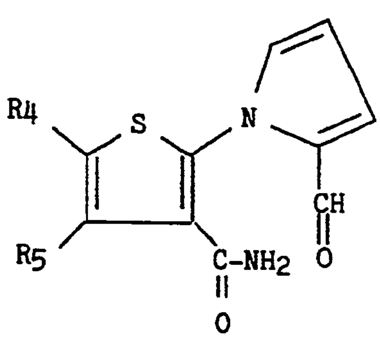

(VII)

dans laquelle $R_4$ et $R_5$ ont la même signification que dans le dérivé de formule générale **(I)**, que l'on fait réagir en suspension dans l'eau avec une amine tertiaire de manière à obtenir le dérivé de formule générale **(VIII)** :

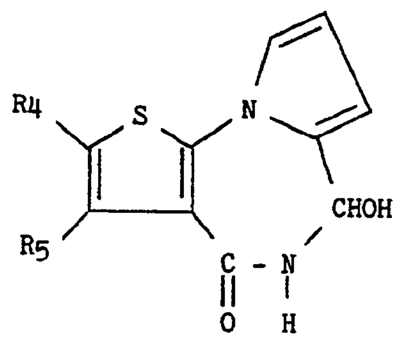

(VIII)

dans laquelle $R_4$ et $R_5$ ont la même signification que dans le dérivé de formule générale **(I)** que l'on réduit en milieu aprotique par un hydrure métallique mixte de manière à obtenir le dérivé de formule générale **(IX)** :

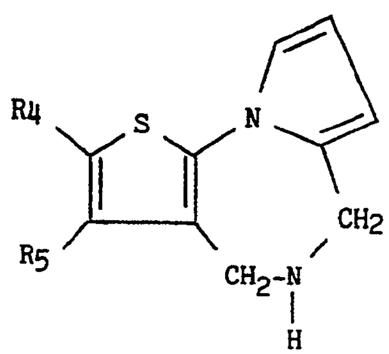

(IX)

dans laquelle $R_4$ et $R_5$ ont la même signification que dans le dérivé de formule générale **(I)** que l'on fait réagir en présence d'amine avec un dérivé halogéné de formule générale **(X)** :

$R_1Cl$ **(X)**

dans laquelle $R_1$ a la même signification que dans le dérivé de formule générale **(I)** de manière à obtenir le dérivé de formule générale **(XI)** :

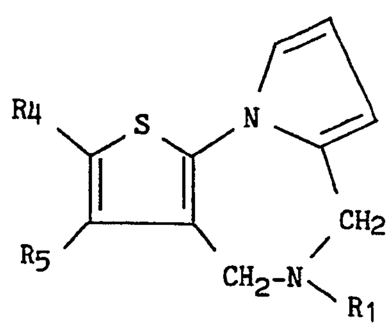

(XI)

dans laquelle $R_1$, $R_4$ et $R_5$ ont la même signification que dans le dérivé de formule générale **(I)**,

- soit l'on part du dérivé de formule générale **(XII)** :

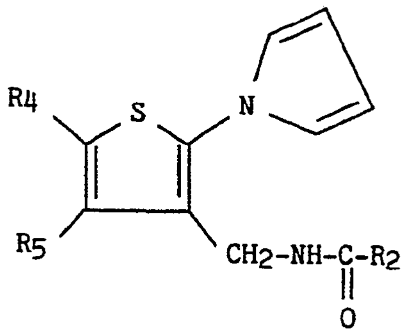

(XII)

dans laquelle $R_2$ ,$R_4$ et $R_5$ ont la même signification que dans le dérivé de formule générale **(I)** que l'on cyclise avec de l'oxychlorure de phosphore de manière à obtenir le dérivé de formule générale **(XIII)** :

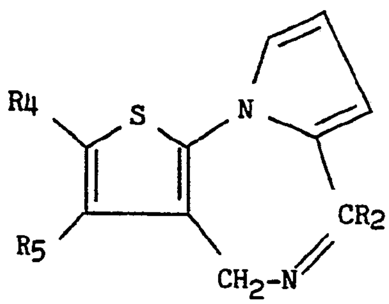

(XIII)

dans laquelle $R_2$, $R_4$ et $R_5$ ont la même signification que dans le dérivé de formule générale **(I)** que l'on réduit en milieu aprotique par un hydrure métallique mixte de manière à obtenir la dihydro-5,6 4H pyrrolo [1,2-a] thiéno [3,2-f] diazépine [1,4] de formule générale **(XIV)** :

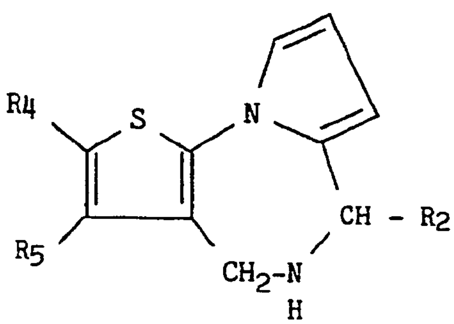

(XIV)

dans laquelle $R_2$, $R_4$ et $R_5$ ont la même signification que dans le dérivé de formule générale **(I)** que l'on peut traiter en solution éthérée par de l'acide chlorhydrique gazeux pour obtenir le chlorure de dihydro-5,6 4H $5H^{\oplus}$ pyrrolo [1,2-a] thiéno [3,2-f] diazepinium [1,4] de formule générale **(XV)**:

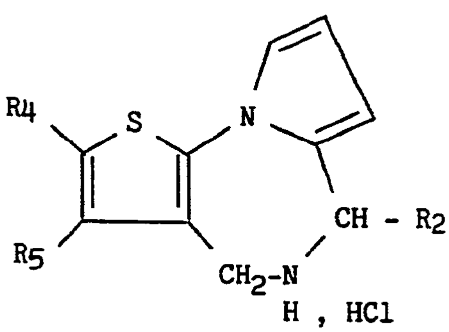

(XV)

dans laquelle $R_2$, $R_4$ et $R_5$ ont la même signification que dans le dérivé de formule générale **(I)**,

- soit l'on part du dérivé de formule générale **(XVI)**:

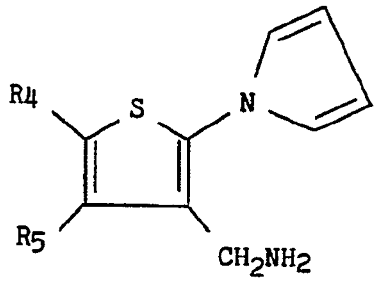

**(XVI)**

dans laquelle $R_4$ et $R_5$ ont la même signification que dans le dérivé de formule générale **(I)** que l'on fait réagir dans de l'alcool au reflux avec un aldéhyde de formule générale **(XVII)**:

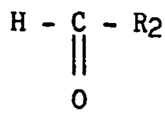

**(XVII)**

dans laquelle $R_2$ a la même signification que dans le dérivé de formule générale **(I)** de manière à obtenir le composé de formule générale **(XVIII)** :

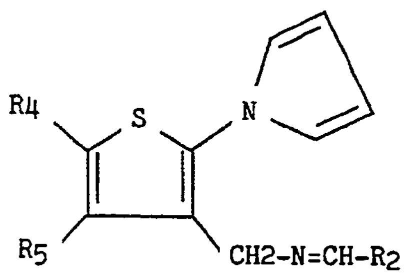

**(XVIII)**

dans laquelle $R_2$,$R_4$ et $R_5$ ont la même signification que dans le dérivé de formule générale **(I)** que l'on traite en solution éthérée par de l'acide chlorhydrique gazeux de manière à obtenir le chlorure de dihydro-5,6 4H 5H⊕ pyrrolo [1,2-a] thiéno [3,2-f] diazépinium [1,4] de formule générale **(XV)** dans laquelle $R_2$, $R_4$ et $R_5$ ont la même signification que dans le dérivé de formule générale **(I)**,

- soit l'on part du dérivé de formule générale **(XIX)**:

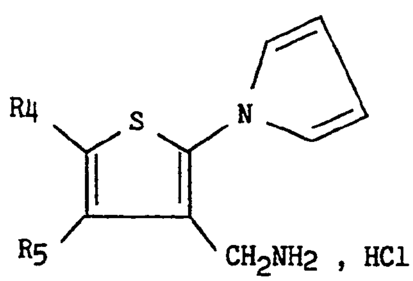

**(XIX)**

dans laquelle $R_4$ et $R_5$ ont la même signification que dans le dérivé de formule générale **(I)** que l'on fait réagir au reflux dans de l'alcool et en présence d'acide chlorhydrique concentré avec une cétone de formule générale **(XX)** :

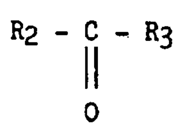

**(XX)**

dans laquelle $R_2$ et $R_3$ ont la même signification que dans le dérivé de formule générale **(I)** de manière à obtenir le chlorure de dihydro-5,6 4H 5H$^{\oplus}$ pyrrolo [1,2-a] thiéno [3,2-f] diazépinium de formule générale **(XXI)** :

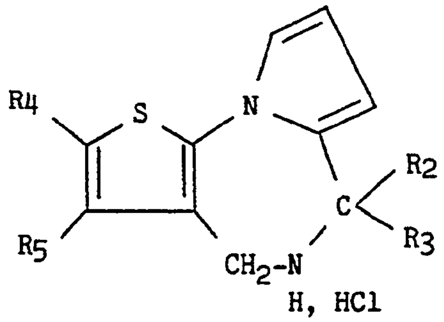

(XXI)

dans laquelle $R_2$, $R_3$, $R_4$ et $R_5$ ont la même signification que dans le dérivé de formule générale **(I)**,

- soit l'on part du dérivé de formule générale **(XIX)** dans laquelle $R_4$ et $R_5$ ont la même signification que dans le dérivé de formule générale **(I)** que l'on fait réagir en milieu alcoolique avec de l'éthoxy-2 hydroxy-2 acétate d'éthyle de manière à obtenir le dérivé de formule générale **(XXII)** :

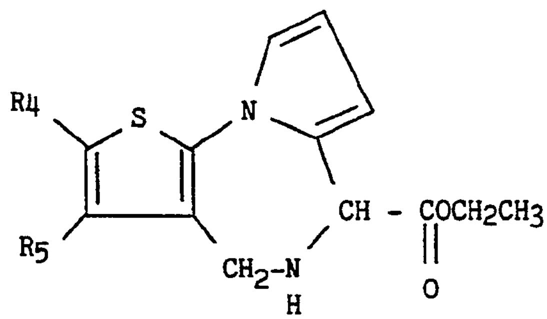

(XXII)

dans laquelle $R_4$ et $R_5$ ont la même signification que dans le dérivé de formule générale **(I)** que l'on réduit en solution alcoolique par un hydrure métallique mixte de manière à obtenir l'hydroxyméthyl-6 dihydro-5,6 4H pyrrolo [1,2-a] thiéno [3,2-f] diazépine [1,4] de formule générale **(XXIII)** :

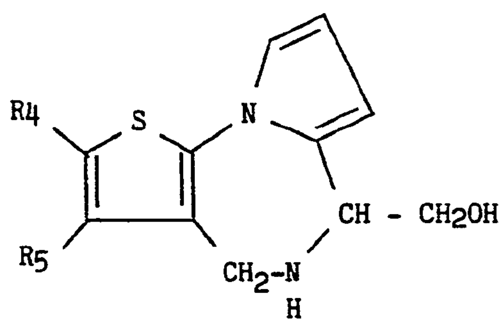

(XXIII)

dans laquelle $R_4$ et $R_5$ ont la même signification que dans le dérivé de formule générale **(I)**,

- soit l'on part du dérivé de formule générale **(VIII)** dans laquelle $R_4$ et $R_5$ ont la même signification que dans le dérivé de formule générale **(I)** que l'on fait réagir au reflux de l'acétonitrile avec une amine de formule générale **(XXIV)** :

HNR'R" **(XXIV)**

dans laquelle R' et R" ont la même signification que dans le dérivé de formule générale **(I)** de manière à obtenir le dérivé de formule générale **(XXV)** :

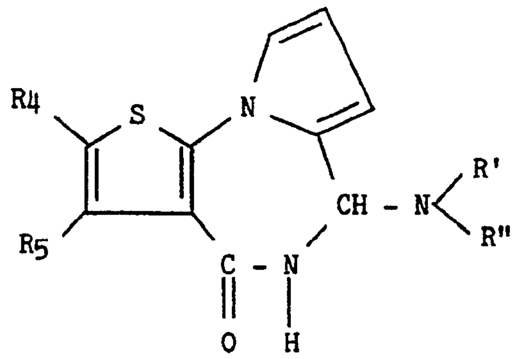

(XXV)

dans laquelle $R_4$, $R_5$, R′ et R″ ont la même signification que dans le dérivé de formule générale **(I)** que l'on réduit en milieu aprotique par un hydrure métallique mixte de manière à obtenir le dérivé de formule générale **(XXVI)** :

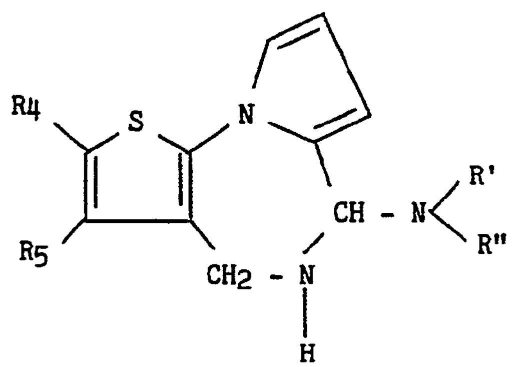

(XXVI)

dans laquelle $R_4$, $R_5$, R′ et R″ ont la même signification que dans le dérivé de formule générale **(I)**.

**2)** Procédé de préparation selon la revendication 1) des composés de formule générale **(I)** pour lesquels $R_4$ et $R_5$ représentent soit un atome d'hydrogène, soit un groupement alkyle inférieur de 1 à 6 atomes de carbone.

**3)** Procédé de préparation selon les revendications 1) et 2) du composé qui est l'(hydroxy-2 pentyl)-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

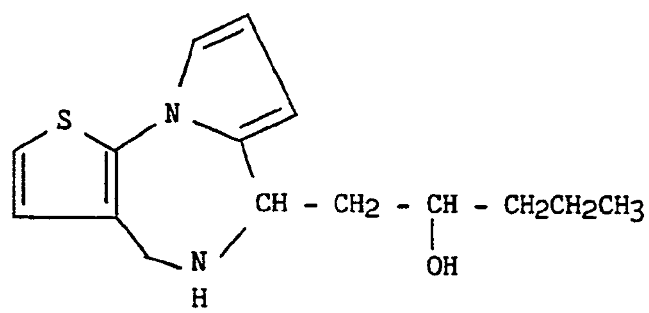

**4)** Procédé de préparation selon les revendications 1) et 2) du composé qui est la (cyclopropyl-2 hydroxy-2 éthyl)-6 dihydro-5,6 4H pyrrolo [1,2-a] thiéno[3,2-f] diazépine [1,4] ainsi que de ses isomères isolés ou sous forme de mélange et de leurs sels d'addition à un acide pharmaceutiquement acceptable.

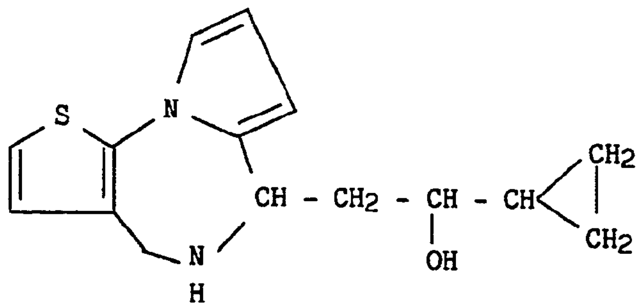

**5)** Procédé de préparation selon les revendications 1) et 2) du composé qui est l'(hydroxy-2 méthyl-3 butyl)-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

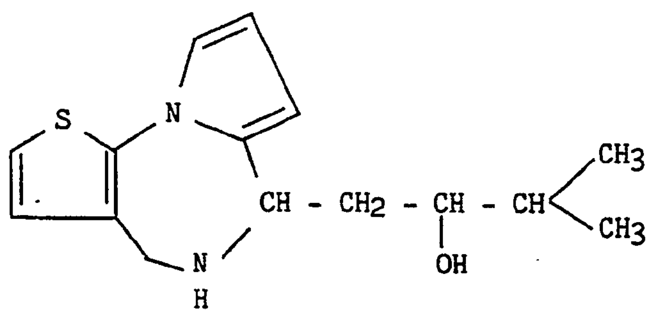

**6)** Procédé de préparation selon les revendications 1) et 2) du composé qui est la (phényl-2 hydroxy-2 éthyl)-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

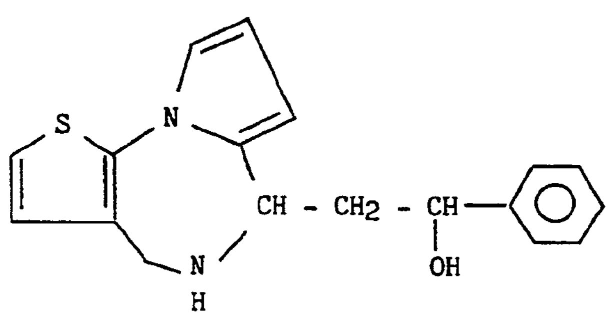

**7)** Procédé de préparation selon les revendications 1) et 2) du composé qui est l'(hydroxy-2 propyl)-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

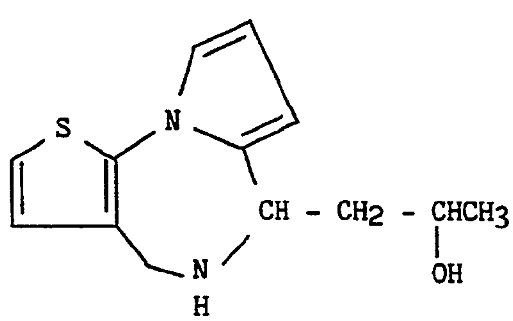

**8)** Procédé de préparation selon les revendications 1) et 2) du composé qui est le para tolyl sulfonyl-5 dihydro-5,6 4H pyrrolo [1,2-a] thiéno [3,2-f] diazépine [1,4].

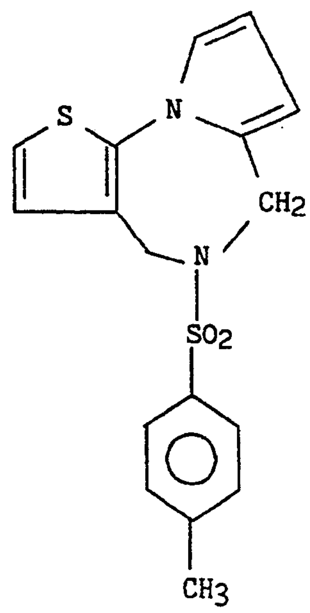

**9)** Procédé de préparation selon les revendications 1) et 2) du composé qui est la diméthyl-6,6 dihydro-5,6 4H pyrrolo [1,2-a] thiéno [3,2-f] diazépine [1,4] ainsi que de ses sels d'addition à un acide pharmaceutiquement acceptable.

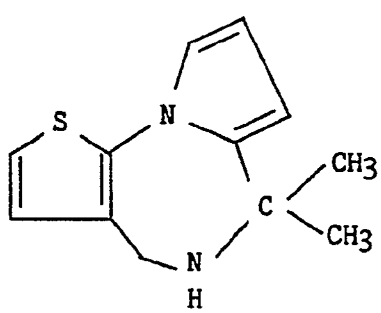

**10)** Procédé de préparation selon les revendications 1) et 2) du composé qui est l'hydroxyméthyl-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

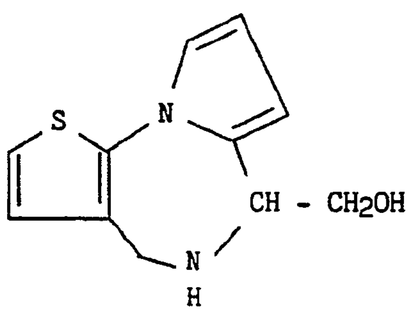

**11)** Procédé de préparation selon les revendications 1) et 2) du composé qui est la cyclopropyl-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

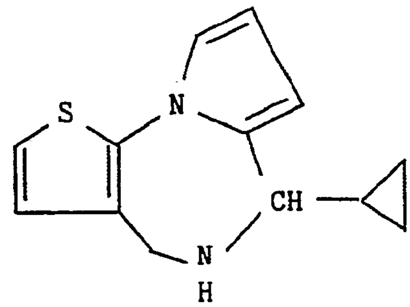

**12)** Procédé de préparation selon les revendications 1) et 2) du composé qui est la méthyl-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

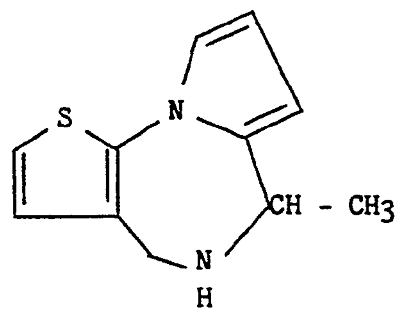

**13)** Procédé de préparation selon les revendications 1) et 2) du composé qui est l'éthyl-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

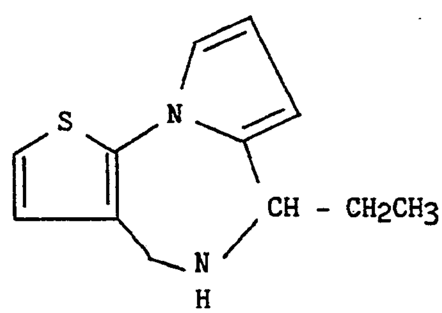

**14)** Procédé de préparation selon les revendications 1) et 2) du composé qui est la diméthyl-2,3 dihydro-5,6 4H pyrrolo [1,2-a] thiéno [3,2-f] diazépine [1,4] ainsi que de ses sels d'addition à un acide pharmaceutiquement acceptable.

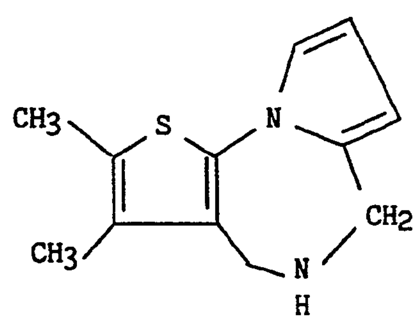

**15)** Procédé de préparation des compositions pharmaceutiques contenant comme principe actif un dérivé préparé selon les revendications 1) à 14) seul ou en combinaison avec un ou plusieurs excipients ou véhicules

inertes non toxiques pharmaceutiquement acceptables.

16) Préparation de compositions pharmaceutiques selon la revendication 15) présentées sous une forme convenant notamment pour le traitement des accidents vasculaires cérébraux, du vieillissement cérébral normal ou pathologique des syndromes ischémiques, de l'hypercholestérolémie de l'hyperlipidémie, de l'hypertriglycéridémie, de l'hyperglycémie, de l'hypertension et des maladies qui leurs sont liées.

**Revendications pour l'Etat contractant suivant : GR**

1) Procédé de préparation de pyrrolo [1,2 -a] thiéno [3,2 -f] diazépine [1,4] de formule générale **(I)** :

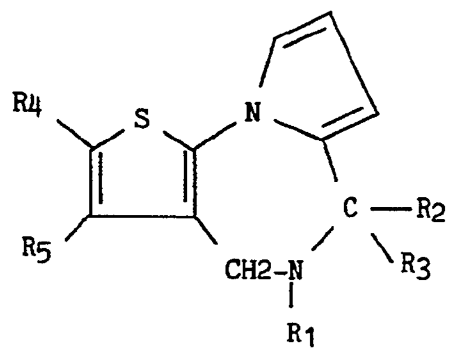

(I)

dans laquelle

– $R_1$ représente un atome d'hydrogène, un groupement alkyle inférieur de 1 à 6 atomes de carbone ramifié ou non éventuellement substitué par des groupements hydroxy, alkoxy, cycloalkyle, aryle, un groupement alkylsulfonyle ou arylsulfonyle éventuellement substitué sur le cycle aromatique par un ou plusieurs groupements alkyles inférieurs ou des halogènes

– $R_4$ et $R_5$ représentent chacun indépendamment l'un de l'autre un atome d'hydrogène, un groupement alkyle inférieur de 1 à 6 atomes de carbone ramifié ou non éventuellement substitué par des groupements hydroxy, alkoxy, cycloalkyle, aryle substitué ou non, un groupement aryle ou arylalkyle éventuellement substitué (le terme substitué associé aux expressions aryle ou aralkyle signifie que les noyaux aromatiques peuvent être substitués par un ou des groupements alkyle, nitro, alkoxy, trifluorométhyle ou halogène)

– $R_2$ et $R_3$ représentent chacun indépendamment l'un de l'autre un atome d'hydrogène, un groupement aryle ou aralkyle éventuellement substitué, un groupement alkyle inférieur de 1 à 6 atomes de carbone ramifié ou non éventuellement substitué par un ou des groupements hydroxy, alkoxy, oxo, cycloalkyle, aryle substitué ou non, ou un groupement -NR'R" pour lequel R' et R" représentent chacun indépendamment l'un de l'autre un atome d'hydrogène, un groupement -COR"' (R"' représente soit un phényl éventuellement substitué, soit un indolyl, soit un groupement anilino éventuellement substitué), un groupement alkyle inférieur, ou forment ensemble avec l'atome d'azote qui les portent un système hétérocyclique mono ou bicyclique comportant ou non un autre hétéroatome et éventuellement substitué par un groupement alkyle inférieur, aryle ou aralkyle éventuellement substitués (le terme substitué associé aux expressions aryle, phényle, anilino et aralkyle signifie que les noyaux aromatiques peuvent être substitués par un ou plusieurs groupements alkyle, nitro, alkoxy, trifluorométhyle ou halogène)

ainsi que de leurs isomères, diastéroisomères, énantiomères et de leur sel d'addition à un acide minéral ou organique, pharmaceutiquement acceptable,
avec la réserve que, lorsque $R_1 = R_4 = R_5 = H$ et l'un des deux substituants $R_2$ ou $R_3$ est un atome d'hydrogène, l'autre substituant ne peut représenter ni un atome d'hydrogène, ni un groupement méthoxy, ni un groupement aryle éventuellement substitué caractérisé en ce que :

- soit l'on part d'un dérivé de formule générale **(II)** :

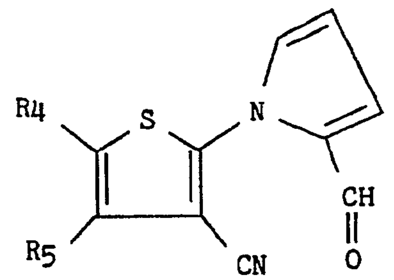

(II)

dans laquelle $R_4$ et $R_5$ ont la même signification que dans le dérivé de formule générale **(I)** que l'on fait réagir dans un mélange d'une solution aqueuse d'hydroxyde de sodium, d'éthanol et de peroxyde d'hydrogène avec une méthyle cétone de formule générale **(III)** :

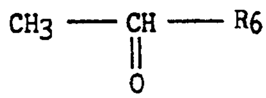

(III)

dans laquelle $R_6$ est un groupement cycloalkyle, alkyle inférieur de 1 à 6 atomes de carbone ramifié ou non éventuellement substitué par des groupements alkoxy, cycloalkyle, aryle, de manière à obtenir la dihydroxy-5,6 oxo-4 4H pyrrolo [1,2-a] thiéno [3,2-f] diazépine [1,4] de formule générale **(IV)** :

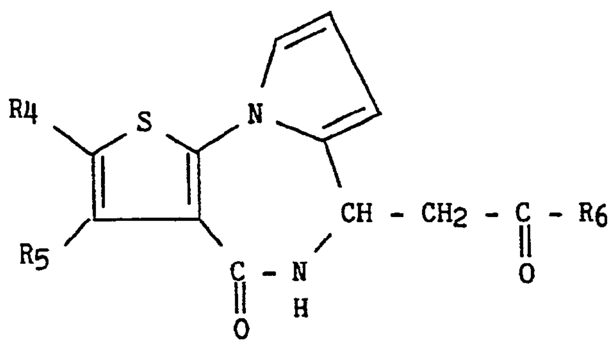

(IV)

dans laquelle $R_6$ a la même signification que dans la formule générale **(III)** et $R_4$ et $R_5$ la même signification que dans le dérivé de formule générale **(I)** que l'on réduit en milieu aprotique par un hydrure métallique mixte de manière à obtenir la dihydro-5,6 4H pyrrolo [1,2-a] thiéno [3,2-f] diazépine [1,4] de formule générale **(V)** :

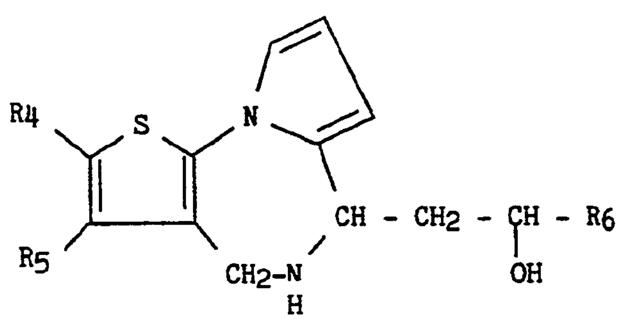

(V)

dans laquelle $R_4$, $R_5$ et $R_6$ ont la même signification que dans la formule **(III)** que l'on peut traiter en solution éthérée par de l'acide chlorhydrique gazeux pour obtenir le chlorure de dihydro-5,6 -4H -5H$^{\oplus}$ pyrrolo [1,2-a] thiéno [3,2-f] diazépinium [1,4] de formule générale **(VI)** :

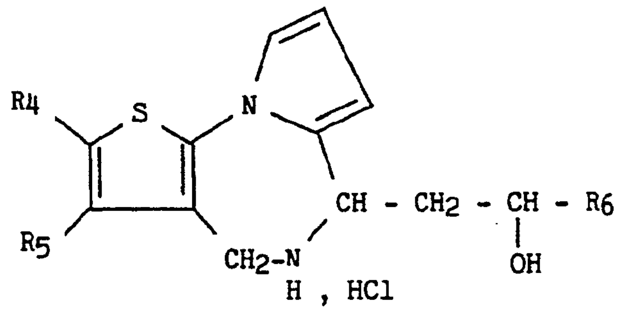

(VI)

dans laquelle $R_4$, $R_5$ et $R_6$ ont la même signification que la formule **(III)**,

- soit l'on part du dérivé de formule générale **(VII)** :

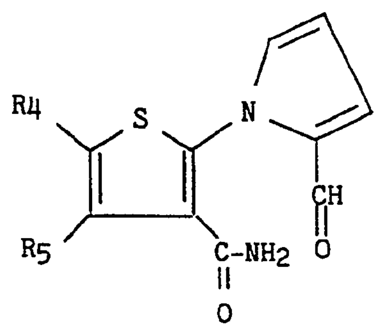

(VII)

dans laquelle $R_4$ et $R_5$ ont la même signification que dans le dérivé de formule générale **(I)**, que l'on fait réagir en suspension dans l'eau avec une amine tertiaire de manière à obtenir le dérivé de formule générale **(VIII)** :

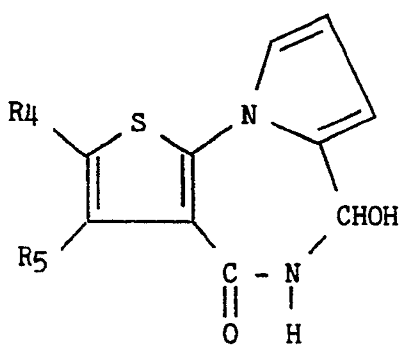

(VIII)

dans laquelle $R_4$ et $R_5$ ont la même signification que dans le dérivé de formule générale **(I)** que l'on réduit en milieu aprotique par un hydrure métallique mixte de manière à obtenir le dérivé de formule générale **(IX)** :

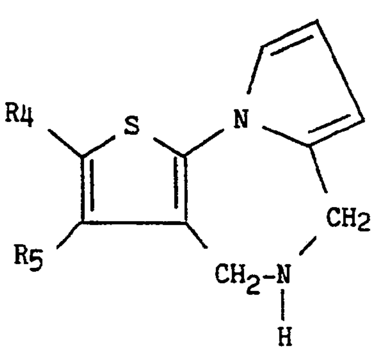

(IX)

dans laquelle $R_4$ et $R_5$ ont la même signification que dans le dérivé de formule générale **(I)** que l'on fait réagir en présence d'amine avec un dérivé halogéné de formule générale **(X)** :

$R_1Cl$ **(X)**

dans laquelle $R_1$ a la même signification que dans le dérivé de formule générale **(I)** de manière à obtenir le dérivé de formule générale **(XI)** :

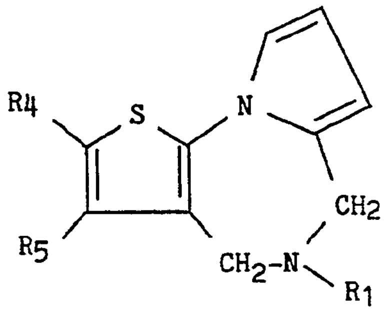

**(XI)**

dans laquelle $R_1$, $R_4$ et $R_5$ ont la même signification que dans le dérivé de formule générale **(I)**,

- soit l'on part du dérivé de formule générale **(XII)** :

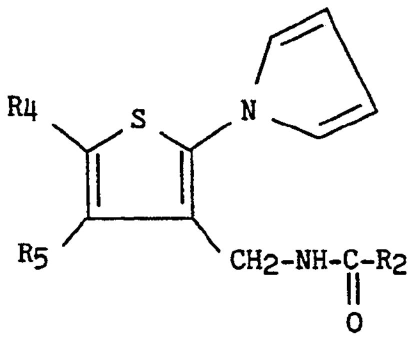

**(XII)**

dans laquelle $R_2$ ,$R_4$ et $R_5$ ont la même signification que dans le dérivé de formule générale **(I)** que l'on cyclise avec de l'oxychlorure de phosphore de manière à obtenir le dérivé de formule générale **(XIII)** :

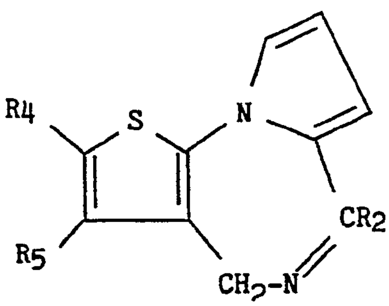

**(XIII)**

dans laquelle $R_2$, $R_4$ et $R_5$ ont la même signification que dans le dérivé de formule générale **(I)** que l'on réduit en milieu aprotique par un hydrure métallique mixte de manière à obtenir la dihydro-5,6 4H pyrrolo [1,2-a] thiéno [3,2-f] diazépine [1,4] de formule générale **(XIV)** :

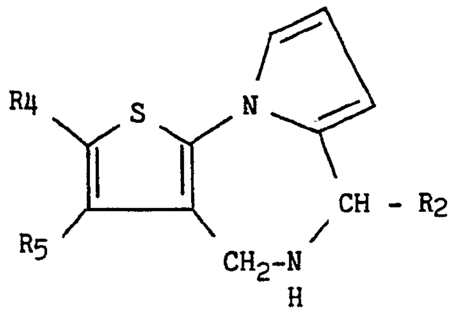

(XIV)

dans laquelle $R_2$, $R_4$ et $R_5$ ont la même signification que dans le dérivé de formule générale **(I)** que l'on peut traiter en solution éthérée par de l'acide chlorhydrique gazeux pour obtenir le chlorure de dihydro-5,6 4H 5H$^{\oplus}$ pyrrolo [1,2-a] thiéno [3,2-f] diazepinium [1,4] de formule générale **(XV)**:

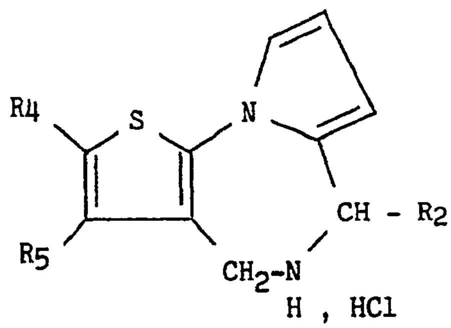

(XV)

dans laquelle $R_2$, $R_4$ et $R_5$ ont la même signification que dans le dérivé de formule générale **(I)**,

- soit l'on part du dérivé de formule générale **(XVI)** :

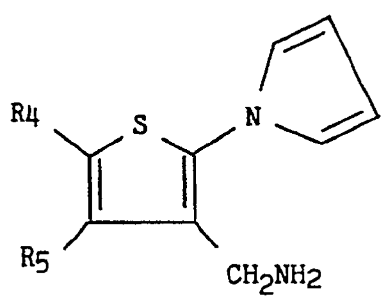

(XVI)

dans laquelle $R_4$ et $R_5$ ont la même signification que dans le dérivé de formule générale **(I)** que l'on fait réagir dans de l'alcool au reflux avec un aldéhyde de formule générale **(XVII)** :

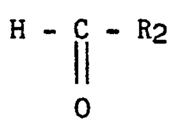

(XVII)

dans laquelle $R_2$ a la même signification que dans le dérivé de formule générale **(I)** de manière à obtenir le composé de formule générale **(XVIII)** :

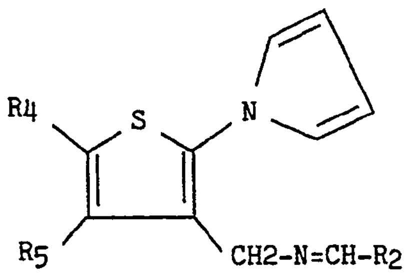

(XVIII)

dans laquelle $R_2$,$R_4$ et $R_5$ ont la même signification que dans le dérivé de formule générale **(I)** que l'on traite en solution éthérée par de l'acide chlorhydrique gazeux de manière à obtenir le chlorure de dihydro-5,6 4H 5H⊕ pyrrolo [1,2-a] thiéno [3,2-f] diazépinium [1,4] de formule générale **(XV)** dans laquelle $R_2$, $R_4$ et $R_5$ ont la même signification que dans le dérivé de formule générale **(I)**,

- soit l'on part du dérivé de formule générale **(XIX)** :

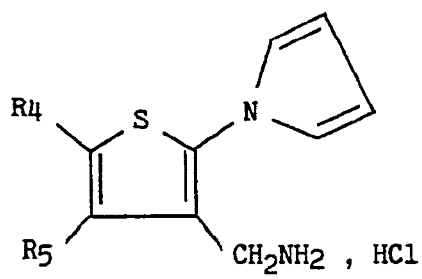

(XIX)

dans laquelle $R_4$ et $R_5$ ont la même signification que dans le dérivé de formule générale **(I)** que l'on fait réagir au reflux dans de l'alcool et en présence d'acide chlorhydrique concentré avec une cétone de formule générale **(XX)** :

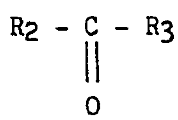

(XX)

dans laquelle $R_2$ et $R_3$ ont la même signification que dans le dérivé de formule générale **(I)** de manière à obtenir le chlorure de dihydro-5,6 4H 5H⊕ pyrrolo [1,2-a] thiéno [3,2-f] diazépinium de formule générale **(XXI)** :

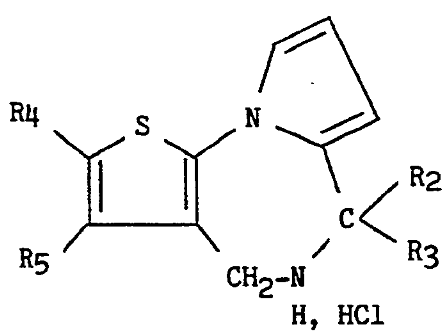

(XXI)

dans laquelle $R_2$, $R_3$, $R_4$ et $R_5$ ont la même signification que dans le dérivé de formule générale **(I)**,

- soit l'on part du dérivé de formule générale **(XIX)** dans laquelle $R_4$ et $R_5$ ont la même signification que dans le dérivé de formule générale **(I)** que l'on fait réagir en milieu alcoolique avec de l'éthoxy-2 hydroxy-2 acétate d'éthyle de manière à obtenir le dérivé de formule générale **(XXII)** :

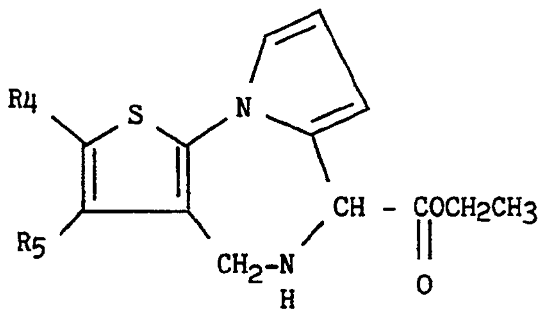

(XXII)

dans laquelle $R_4$ et $R_5$ ont la même signification que dans le dérivé de formule générale **(I)** que l'on réduit en solution alcoolique par un hydrure métallique mixte de manière à obtenir l'hydroxyméthyl-6 dihydro-5,6 4H pyrrolo [1,2-a] thiéno [3,2-f] diazépine [1,4] de formule générale **(XXIII)** :

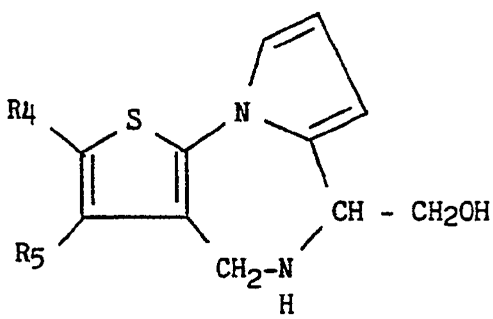

(XXIII)

dans laquelle $R_4$ et $R_5$ ont la même signification que dans le dérivé de formule générale **(I)**,

- soit l'on part du dérivé de formule générale **(VIII)** dans laquelle $R_4$ et $R_5$ ont la même signification que dans le dérivé de formule générale **(I)** que l'on fait réagir au reflux de l'acétonitrile avec une amine de formule générale **(XXIV)** :

HNR'R″ **(XXIV)**

dans laquelle R' et R″ ont la même signification que dans le dérivé de formule générale **(I)** de manière à obtenir le dérivé de formule générale **(XXV)** :

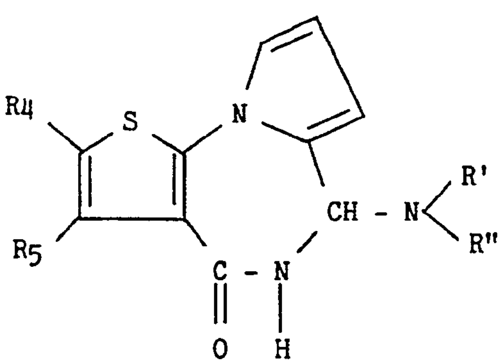

(XXV)

dans laquelle $R_4$, $R_5$, R' et R″ ont la même signification que dans le dérivé de formule générale **(I)** que l'on réduit en milieu aprotique par un hydrure métallique mixte de manière à obtenir le dérivé de formule générale **(XXVI)** :

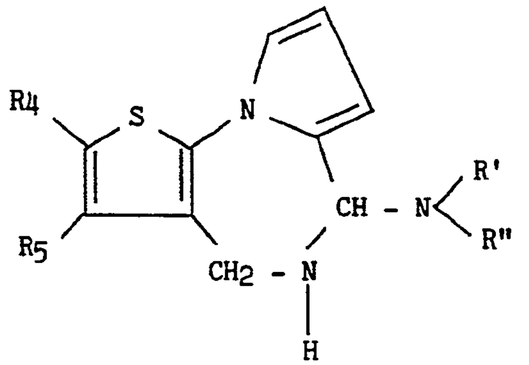

(XXVI)

dans laquelle $R_4$, $R_5$, R' et R" ont la même signification que dans le dérivé de formule générale **(I)**.

**2)** Procédé de préparation selon la revendication 1) des composés de formule générale **(I)** pour lesquels $R_4$ et $R_5$ représentent soit un atome d'hydrogène, soit un groupement alkyle inférieur de 1 à 6 atomes de carbone.

**3)** Procédé de préparation selon les revendications 1) et 2) du composé qui est l'(hydroxy-2 pentyl)-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

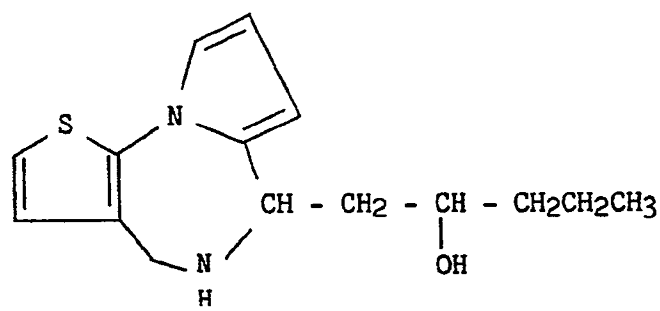

**4)** Procédé de préparation selon les revendications 1) et 2) du composé qui est la (cyclopropyl-2 hydroxy-2 éthyl)-6 dihydro-5,6 4H pyrrolo [1,2-a] thiéno[3,2-f] diazépine [1,4] ainsi que de ses isomères isolés ou sous forme de mélange et de leurs sels d'addition à un acide pharmaceutiquement acceptable.

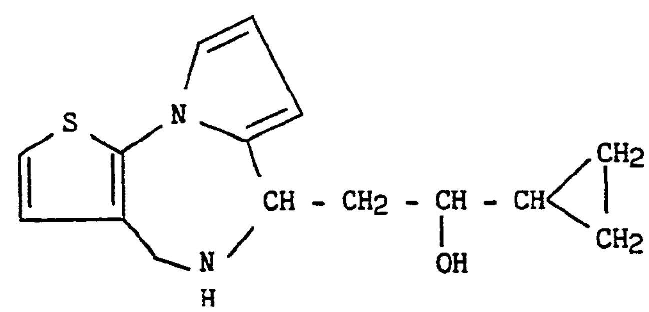

**5)** Procédé de préparation selon les revendications 1) et 2) du composé qui est l'(hydroxy-2 méthyl-3 butyl)-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

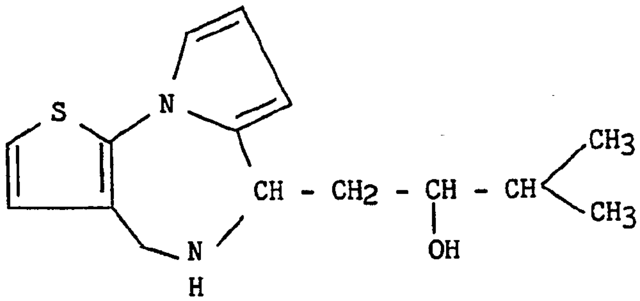

**6)** Procédé de préparation selon les revendications 1) et 2) du composé qui est la (phényl-2 hydroxy-2 éthyl)-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

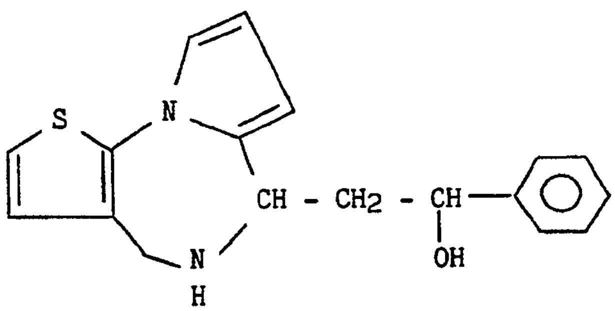

**7)** Procédé de préparation selon les revendications 1) et 2) du composé qui est l'(hydroxy-2 propyl)-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

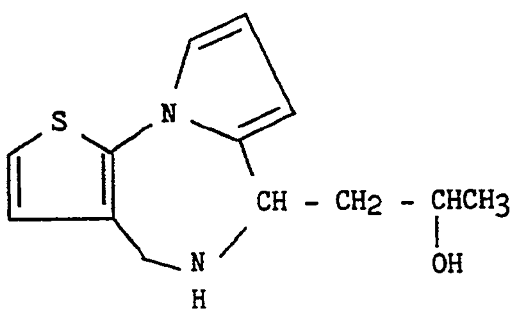

**8)** Procédé de préparation selon les revendications 1) et 2) du composé qui est le para tolyl sulfonyl-5 dihydro-5,6 4H pyrrolo [1,2-a] thiéno [3,2-f] diazépine [1,4].

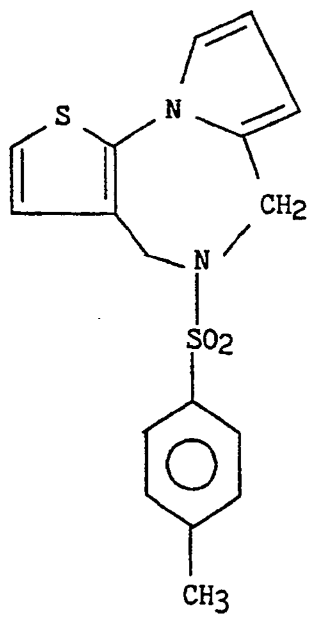

**9)** Procédé de préparation selon les revendications 1) et 2) du composé qui est la diméthyl-6,6 dihydro-5,6 4H pyrrolo [1,2-a] thiéno [3,2-f] diazépine [1,4] ainsi que de ses sels d'addition à un acide pharmaceutiquement acceptable.

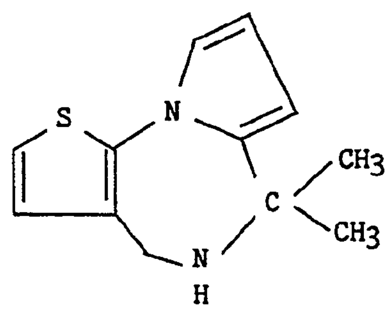

**10)** Procédé de préparation selon les revendications 1) et 2) du composé qui est l'hydroxyméthyl-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

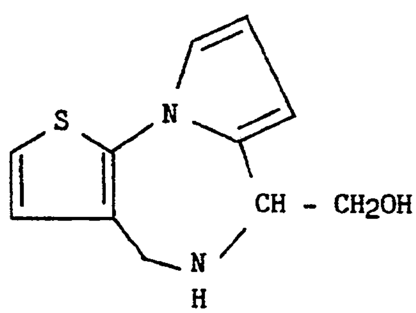

**11)** Procédé de préparation selon les revendications 1) et 2) du composé qui est la cyclopropyl-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

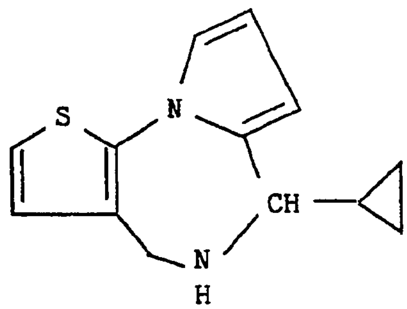

**12)** Procédé de préparation selon les revendications 1) et 2) du composé qui est la méthyl-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

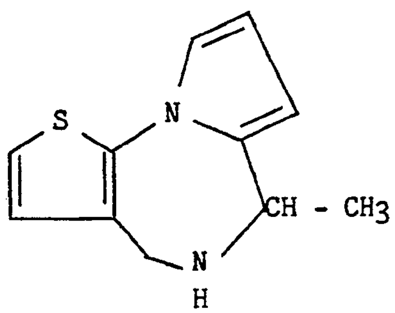

**13)** Procédé de préparation selon les revendications 1) et 2) du composé qui est l'éthyl-6 dihydro-5,6 4H pyrrolo [1,2-a] thiéno [3,2-f] diazépine [1,4] ainsi que de ses isomères isolés ou sous forme de mélange et de leurs sels d'addition à un acide pharmaceutiquement acceptable.

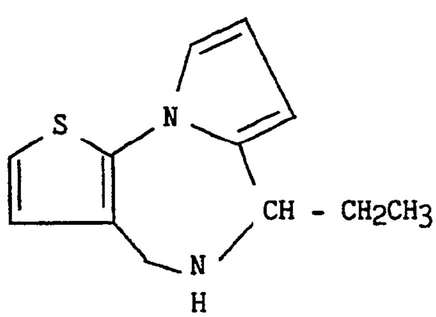

**14)** Procédé de préparation selon les revendications 1) et 2) du composé qui est la diméthyl-2,3 dihydro-5,6 4H pyrrolo [1,2-a] thiéno [3,2-f] diazépine [1,4] ainsi que de ses sels d'addition à un acide pharmaceutiquement acceptable.

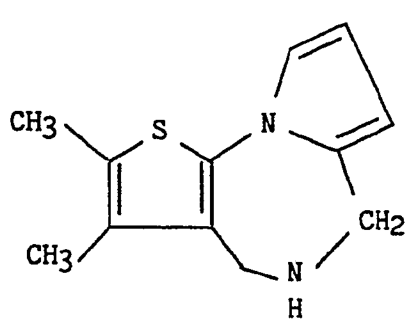

**15)** Procédé de préparation des compositions pharmaceutiques contenant comme principe actif un dérivé préparé selon les revendications 1) à 14) seul ou en combinaison avec un ou plusieurs excipients ou véhicules

inertes non toxiques pharmaceutiquement acceptables.

**16)** Préparation de compositions pharmaceutiques selon la revendication 15) présentées sous une forme convenant notamment pour le traitement des accidents vasculaires cérébraux, du vieillissement cérébral normal ou pathologique des syndromes ischémiques, de l'hypercholestérolémie de l'hyperlipidémie, de l'hypertriglycéridémie, de l'hyperglycémie, de l'hypertension et des maladies qui leurs sont liées.

Office européen des brevets

# RAPPORT DE RECHERCHE EUROPEENNE

Numero de la demande

EP 91 40 0644
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | Comptes Rendus Hebd. des Séances de l'Académie des Sciences, Série C, vol. 285, no. 11, 31 octobre 1977, Paris, pages 381 - 383; S. RAULT et al.: "Etude des conditions d'accès aux 4H-dihydro-5.6-pyrrolo[1.2-a]thiéno[3.2-f]diazépines-1.4" * page 381 * | 1, 2, 15 | C07D495/14<br>A61K31/55<br>//(C07D495/14, 333:00,207:00, C07D243:00) |
| A | CHEMICAL ABSTRACTS, vol. 90, no. 3, 15 janvier 1979 Columbus, Ohio, USA S.RAULT et al.: "Synthesis and study of 4H-5,6-dihydro[1,2-a]pyrrolo[3,2-f]thieno-1,4-diazepine" page 650; ref. no. 22995P & C. R. Hebd. Seances Acad. Sci., Ser. C 1978, vol. 287, no. 4, pages 117-120 * abrégé * | 1, 2, 15 | |
| A | CHEMICAL ABSTRACTS, vol. 87, no. 11, 12 septembre 1977 Columbus, Ohio, USA S. RAULT et al.: "Study of conditions for access to 1H-thieno[3,2-f]pyrrolo[1,2-a][1,4]diazepines" page 596; ref. no. 84963Y & C. R. Hebd. Seances Acad. Sci., Ser. C 1977, vol. 284, no. 14, pages 533-535 * abrégé * | 1, 2, 15 | **DOMAINES TECHNIQUES RECHERCHES (Int. Cl.5)**<br>C07D495/00 |
| A | CHEMICAL ABSTRACTS, vol. 93, no. 7, 18 août 1980 Columbus, Ohio, USA S. RAULT et al.: "Synthesis of 4H-6-aminopyrrolo[1,2-a]thieno[3,2-f]-1,4-diazepine" page 976; ref. no. 71726X & C. R. Hebd. Seances Acad. Sci., Ser. C 1980, vol. 290, no. 9, pages 169-171 * abrégé * | 1, 2, 15 | |
| | -/-- | | |

Le présent rapport a été établi pour toutes les revendications

| Lieu de la recherche | Date d'achèvement de la recherche | Examinateur |
|---|---|---|
| BERLIN | 10 JUIN 1991 | HASS C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 91 40 0644
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | CHEMICAL ABSTRACTS, vol. 92, no. 7, 18 février 1980<br>Columbus, Ohio, USA<br>S. RAULT et al.: "Novel convenient synthesis of 1,4-diazepines; 6-alkoxy-5,6-dihydro-4H-pyrrolo[1,2-a]thieno[3.2-f]-1,4-diazepin-4-ones"<br>page 672; ref. no. 58739P & Heterocycles 1979, vol. 12, no. 8, pages 1009-1011<br>* abrégé *<br>--- | 1, 2, 15 | |
| A | CHEMICAL ABSTRACTS, vol. 88, no. 7, 13 février 1978<br>Columbus, Ohio, USA<br>page 556; ref. no. 50934V<br>& JP-A-77 100496 [cat. D]<br>* abrégé *<br>--- | 1, 2, 15 | |
| A | EP-A-0230942 (BOEHRINGER INGELHEIM KG)<br>* revendications 1, 8, 10 *<br>--- | 1, 16, 17 | |
| A | EP-A-0240899 (BOEHRINGER INGELHEIM KG)<br>* revendications 1, 8, 10 *<br>--- | 1, 16, 17 | DOMAINES TECHNIQUES RECHERCHES (Int. Cl.5 ) |
| A | EP-A-0342587 (YOSHITOMI PHARMACEUTICAL INDUSTRIES, LTD.)<br>* revendications 1, 3, 4 *<br>----- | 1, 16, 17 | |

Le présent rapport a été établi pour toutes les revendications

| Lieu de la recherche | Date d'achèvement de la recherche | Examinateur |
|---|---|---|
| BERLIN | 10 JUIN 1991 | HASS C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)